# EUROPEAN PATENT APPLICATION

(11) **EP 1 411 457 A2**
(43) Date of publication of application: **21.04.2004**
(21) Application number: 03022857.1
(22) Date of filing: 08.10.2003
(51) Int. Cl.: G06F 19/00

(54) **Diagnosis report generation system**

(30) Priority: 17.10.2002 JP 2002302813
(71) Applicant: Olympus Corporation, Shibuya-ku, Tokyo (JP)
(72) Inventor: Tatsuya, Shiobara, Hachioji-shi Tokyo 192-0045 (JP)
(74) Representative: von Hellfeld, Axel, Dr. Dipl.-Phys.

(57) **Abstract**

An observation display unit displays observations on an examination result. In response to the display of the observations by the observation display unit, a treatment information input request notification unit issues a notification of a request to input treatment information about the treatment relating to the observation. Upon receipt of the above-mentioned notification, a treatment information obtaining unit obtains input treatment information. A storage unit associates observation information about the observation with the above-mentioned treatment information, and stores it.

## Description

### Cross Reference to the related application

This application is based upon and claims the benefit of priority from the prior Japanese Application No. 2002-302813, filed Oct. 17, 2002, the entire contents of which are incorporated herein by reference.

### Background of the Invention

### Field of the Invention

The present invention relates to the technology of supporting medical practice, and more specifically to the technology of generating and managing a diagnosis report in which the states of implementation of examinations, the observations of doctors on examination results, etc. are summarized.

### Description of the Related Art

Japanese Patent Application Laid-open No.2002-73615 discloses the invention of a medical image filing system for management of profile data of patients, reservation information about examinations, result information, etc. in addition to recording, retrieving, and generating images. In this medical image filing system, examination reports (diagnosis reports) can be managed. A diagnosis report practically refers to observations, comments, etc. on the state of implementation of examinations, and is input by a doctor.

Conventionally, a diagnosis report is hand-written by a doctor, but the diagnosis report managed by the above-mentioned medical image filing system is prepared by the combination of the display of a screen and the operation of a keyboard. In preparing the diagnosis report managed by the medical image filing system, the description order of items, the terms, etc. largely depend on the style of the doctor who prepares the report.

### Summary of the Invention

As an aspect of the present invention, the diagnosis report generation system includes an observation display unit for displaying observations on an examination result, a treatment information input request notification unit for transmitting a notification of a request to input treatment information about the treatment in response to the observation when the observation is displayed on the observation display unit, a treatment information obtaining unit for obtaining the treatment information input in response to the notification, and a storage unit for storing observation information indicating the observation associated with the treatment information.

With the above-mentioned configuration, it is desired that the system also includes an observation term selection result obtaining unit for obtaining a selection result of an observation term corresponding to the observation from a plurality of observation terms, and a supplementary information result obtaining unit for obtaining the selection result of supplementary information corresponding to the observation from plural pieces of supplementary information associated with the observation term relating to the selection result so that the observation information can be formed by the observation terms relating to the selection result about the observation term and the supplementary information relating to the selection result about the supplementary information.

Furthermore, it is desired that the system also includes an observation term list display unit for displaying a list of a plurality of observation terms.

Additionally, it is desired that the system includes a supplementary information list display unit for displaying a list of supplementary information associated with the observation term so that the supplementary information result obtaining unit can obtain the selection result from the list of the supplementary information.

Furthermore, it is desired that the supplementary information list display unit can display a list of the supplementary information based on the selection result of the observation term obtained by the observation term selection result obtaining unit.

Additionally, it is desired that the observation display unit can display a list of observation terms relating to the selection result about the observation term and the supplementary information relating to the selection result about the supplementary information.

Furthermore, it is desired that the system includes an observation text generation unit for generating observation text representing the observation term relating to the selection result and the supplementary information relating to the selection result about the supplementary information so that the observation display unit can display the observation text.

It is also desired that the system further includes an observation display format switch unit for switching between the display of the observation on the observation display unit represented as a list of observation terms relating to the selection result about the observation term and supplementary information relating to the selection result about the supplementary information, and the display of the observation text.

According to another aspect of the present invention, the diagnosis report generation system also includes an observation term selection result obtaining unit for obtaining a selection result from a plurality of observation terms about an observation term corresponding to observation on an examination result, a supplementary information result obtaining unit for obtaining the selection result of supplementary information corresponding to the observation from plural pieces of supplementary information associated with the observation term relating to the selection result, and an observation information storage unit for storing observation information which is formed by the observation term relating to the selection result about an observation term and the supplementary information relating to the selection result about the supplementary information, and indicates the observation.

### Brief Description of the Drawings

The present invention will be more apparent from the following detailed description when the accompanying drawings are referenced.
FIG. 1 shows the configuration representing the principle of the diagnosis report generation system embodying the present invention;
FIG. 2A shows a practical configuration of the diagnosis report generation system embodying the present invention;
FIG. 2B shows the configuration of each of the server devices and the terminal devices shown in FIG. 2A;
FIG. 3 shows the structure of the database of the server device;
FIGS. 4A and 4B are flowcharts of the processes of a report window process;
FIG. 5 shows an example of a report window screen;
FIGS. 6A and 6B are flowcharts of the processes of a new observation input process;
FIG. 7 shows a display example of a list of observation terms;
FIG. 8 shows a display example of a list of various attributes of observation terms and target particular regions;
FIG. 9 shows a display example of determined observation;
FIG. 10 shows a display example of a list of various attributes corresponding to an observation term and target particular regions in a text mode;
FIG. 11 shows a display example of determined observation in a text mode;
FIGS. 12A and 12B are flowcharts showing the contents of the new observation input process;
FIG. 13 shows a display example of a list of treatment terms;
FIG. 14 shows a display example of a window screen having a list of various attributes corresponding to treatment terms and input columns of specimen numbers;
FIG. 15 shows an example of a numeric input window screen for input of a specimen number;
FIG. 16 shows a display example of observation and treatment after determining the input of treatment;
FIG. 17 is a flowchart of the process of a new diagnosis input process;
FIG. 18 shows a display example of a list of diagnosed regions;
FIG. 19 shows a display example of a list of diagnostic terms;
FIG. 20 shows a display example of a list of various attributes and a list of predicates corresponding to diagnostic terms;
FIG. 21 shows a display example of a list of predicates corresponding to diagnostic terms;
FIG. 22 shows a display example after a new diagnosis input;
FIGS. 23A and 23B are flowcharts of a new comment input process;
FIG. 24 shows a display example of a list of items to be commented on;
FIG. 25 shows a display example of a window screen having a report comment input area;
FIG. 26 shows a display example of a window screen having a pathological order comment input area;
FIG. 27 shows a display example of a window screen having a post-examination instruction input area;
FIG. 28 shows a display example after determination of comment input;
FIGS. 29A and 29B are flowcharts of an existing observation editing/deleting process;
FIG. 30 shows a display example of a list of edit instruction buttons for use during the editing of an observation;
FIG. 31 shows a display example of a list of various attributes and target particular regions corresponding to the observation term during the editing of an observation;
FIG. 32 shows a display example after a part of an observation has been deleted;
FIGS. 33A and 33B are flowcharts of an existing observation editing/deleting process;
FIG. 34 shows a display example of a list of edit instruction buttons for use during the editing of a treatment;
FIG. 35 shows a display example of a window screen having a list of various attributes and input columns of specimen numbers corresponding to the treatment term during the editing of a treatment;
FIG. 36 shows a display example after a part of treatment has been deleted;
FIG. 37 is a flowchart of an existing diagnosis editing/deleting process;
FIG. 38 shows a display example of a list of predicates corresponding to the diagnostic term during the editing of diagnosis;
FIG. 39A is a flowchart of an existing comment editing/deleting process;
FIG. 39B is a flowchart of a comment deleting/changing process;
FIG. 40 shows a display example of a list of edit instruction buttons for use during the editing of comments;
FIG. 41 shows a display example after a part of a comment has been deleted;
FIG. 42A is a flowchart of an observation representation format switching process;
FIG. 42B is a flowchart of an existing observation representation format switching process;
FIG. 42C is a flowchart of a representation format switching process; and
FIG. 43 shows an example of a recording medium storing a computer-readable control program.

### Description of the Preferred Embodiments

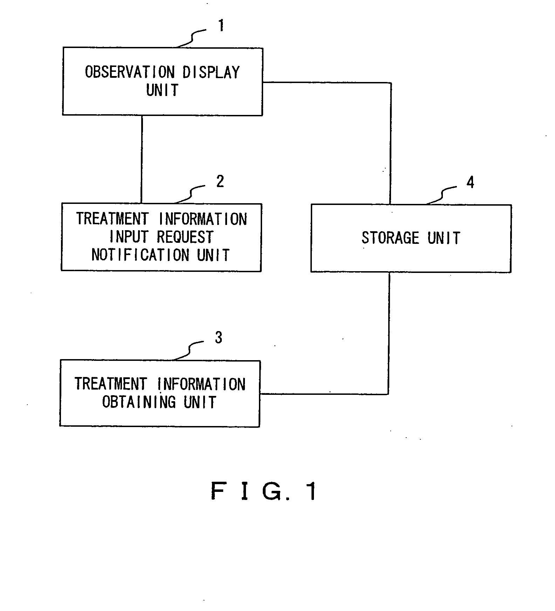

Explanation of FIG. 1 is given first below. FIG. 1 shows the configuration representing the principle of the diagnosis report generation system embodying the present invention.

An observation display unit 1 displays the observation on an examination result.

In response to the display of the observation on the observation display unit 1, a treatment information input request notification unit 2 issues a notification of a request to input treatment information about the treatment corresponding to the observation.

A treatment information obtaining unit 3 obtains treatment information input depending on the above-mentioned notification.

A storage unit 4 stores observation information indicating the above-mentioned observation associated with the above-mentioned treatment information.

With the above-mentioned configuration, observation is followed by the input of a corresponding treatment, that is, a request notification to input treatment by reflecting a general procedure of generating a diagnosis report. Therefore, the interference with the normal flow of thought by the investigation of the treatment after the observation can be eliminated. Therefore, an uncomfortable feeling of a report maker in hand-writing a diagnosis report can be avoided.

The diagnosis report generation system for embodying the present invention shown in FIG. 1 can also be designed to comprise the observation term selection result obtaining unit for obtaining a selection result of an observation term corresponding to the above-mentioned observation from a plurality of observation terms, and the supplementary information result obtaining unit for obtaining a selection result of supplementary information corresponding to the observation from plural pieces of supplementary information associated with the observation term relating to the selection result so that the observation information can be formed by an observation term relating to the selection result about an observation term and supplementary information relating to the selection result about supplementary information.

With the above-mentioned configuration, the contents of observation are represented by a term selected from a plurality of predetermined terms. Therefore, the variance of representation of the contents depending on the report makers can be reduced. As a result, a person other than the report maker can easily understand the diagnosis report. Furthermore, the information contained in a diagnosis report can be easily stored in a database for secondary use of the information.

At this time, the system can further comprise the observation term list display unit for displaying a list of a plurality of observation terms.

With this configuration, a list of selectable observation terms for description of the contents of observation is presented. Therefore, a diagnosis report maker can easily describe his or her observation.

The system can further comprise the supplementary information list display unit for displaying a list of supplementary information associated with observation terms so that the supplementary information result obtaining unit can obtain a selection result from the list of supplementary information.

With this configuration, a list of selectable supplementary information for description of the contents of observation is presented. Therefore, a diagnosis report maker can easily describe his or her observation.

At this time, the supplementary information list display unit can also be designed to display a list of supplementary information based on the selection result of an observation term obtained by the above-mentioned observation term selection result obtaining unit.

With this configuration, a list of supplementary information only relating to the selected observation term can be presented. Therefore, a diagnosis report maker can easily select desired supplementary information.

In the diagnosis report generation system which embodies the present invention shown in FIG. 1, the observation display unit 1 can be configured to display a list of an observation term relating to the selection result about an observation term and supplementary information relating to the selection result about supplementary information.

With this configuration, the contents of an observation can be briefly displayed.

Furthermore, the diagnosis report generation system which embodies the present invention shown in FIG. 1 can further comprise an observation term list display unit for generating observation text formed by the above-mentioned observation term relating to the selection result and the above-mentioned supplementary information relating to the selection result about the supplementary information, and the observation display unit 1 can be designed to display the observation text.

With this configuration, the contents of an observation term are represented by text. Therefore, it is helpful for a patient, etc. having little medical knowledge to understand the contents of an observation.

At this time, the system can also comprise an observation display format switch unit for switching between the display of the observation on the observation display unit represented as a list of observation terms relating to the selection result about the observation term and supplementary information relating to the selection result about the supplementary information, and the display of the observation text.

With this configuration, the display of the contents of observation can be switched as necessary between simple representation and text representation.

The system can also comprise the observation term selection result obtaining unit for obtaining a selection result from a plurality of observation terms about an observation term corresponding to observation on an examination result, the supplementary information result obtaining unit for obtaining the selection result of supplementary information corresponding to the observation from plural pieces of supplementary information associated with the observation term relating to the selection result, and the observation information storage unit for storing observation information which is formed by the observation term relating to the selection result about an observation term and the supplementary information relating to the selection result about the supplementary information, and indicates the observation.

With the above-mentioned configuration, the contents of observation are represented by a term selected from a plurality of predetermined terms. Therefore, the variance of representation of the contents depending on the report makers can be reduced. As a result, a person other than the report maker can easily understand the diagnosis report. Furthermore, the information contained in a diagnosis report can be easily stored in a database for secondary use of the information.

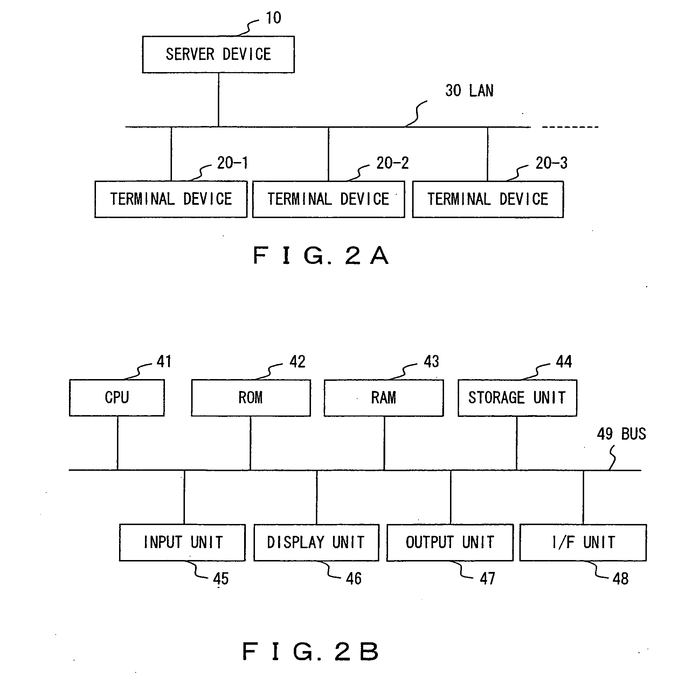

The components shown in FIGS. 2A and 2B are described below.

FIG. 2A shows a practical configuration of the diagnosis report generation system embodying the present invention. In this system, a server device 10 storing various data forming a generated diagnosis report is connected to terminal devices 20-1, 20-2, and 20-3 operated for generation, browsing, and output of the diagnosis report through a LAN (local area network) 30 so that various data can be communicated among the terminal devices 20-1, 20-2, and 20-3.

Although FIG. 2A shows the three terminal devices 20-1, 20-2, and 20-3, the number of terminal devices connected to the LAN 30 can be arbitrarily set.

FIG. 2B shows the hardware configuration of each of the server device 10 and the terminal devices 20-1, 20-2, and 20-3.

The device shown in FIG. 2B is configured by a CPU 41, ROM 42, RAM 43, an HDD 44, an input unit 45, a display unit 46, an output unit 47, and an I/F unit 48 mutually connected through a bus 49 such that data can be communicated under the management of the CPU 41.

The CPU 41 is a central processing unit for controlling the operation of the entire device shown in FIG. 2B.

The ROM (read only memory) 42 is memory storing in advance a basic control program. When the device is activated, the CPU 41 executes the control program to allow the basic control of the operation of the entire device to be performed by the CPU 41.

The RAM (random access memory) 43 is memory to be used as work memory when each control program is executed by the CPU 41, and also functions as main memory used as necessary as a temporary storage area of various data.

The storage unit 44 is configured by providing, for example, an HDD (hard disk drive). When the device shown in FIG. 2B is used as any of the terminal devices 20-1, 20-2, and 20-3, the storage unit 44 stores in advance a control program for allowing the CPU 41 to perform the processes of generating, editing, and recording a diagnosis report described later. When the device shown in FIG. 2B is used as the server device 10, the storage unit 44 functions as a data storage device of a database storing various data forming a diagnosis report, and also stores in advance a control program for allowing the CPU 41 to perform a data manipulating process on the database at various data manipulate instructions from the terminal devices 20-1, 20-2, and 20-3.

The input unit 45 receives externally input data and transmits the contents of the input data to the CPU 41, and is provided with, for example, an input device for receiving an instruction from an operator who operates the device. The input unit 45 can also be configured by including as necessary a reader of a portable storage medium such as an FD (flexible disk), CD-ROM (compact disk-ROM), DVD-ROM (digital versatile disk-ROM), an MO (magneto-optics) disk, etc.

The display unit 46 displays various information at an instruction from the CPU 41, and is configured by including, for example, a CRT (cathode ray tube), an LCD (liquid crystal display), etc.

The output unit 47 outputs various information at an instruction from the CPU 41, and can be, for example, a printer device for printing a diagnosis report, etc.

The I/F (interface) unit 48 connects the device to the LAN 30 shown in FIG. 2A, and manages the communications of data with other devices.

Since the device shown in FIG. 2B is normally included in the computer system with a standard hardware configuration, the server device 10 and the terminal devices 20-1, 20-2, and 20-3 can be configured using such a computer system.

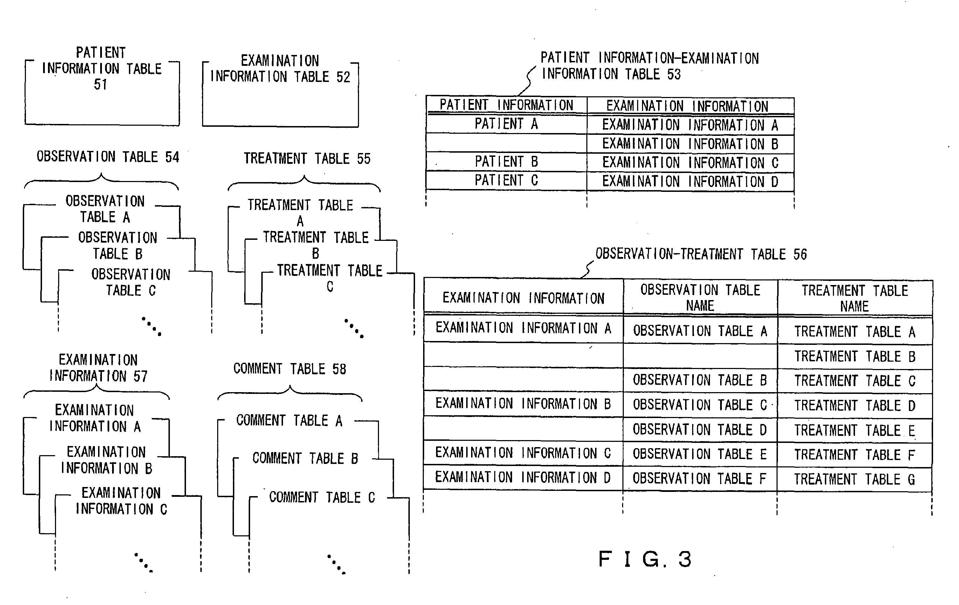

Described below is the configuration shown in FIG. 3. FIG. 3 shows the structure of the database provided for the server device 10.

A patient information table 51 shows data by patient indicating the patient information about the ID (identification) number, sex, birthday, age, type (inpatient, outpatient, etc.), etc. assigned to each patient.

An examination information table 52 shows data in an examination unit indicating an examination type, an examination item, an assumed illness, the date and time of making an examination, a requested department for an examination, the name of a requested doctor, etc.

A patient information-examination information table 53 shows the relationship between the patient information shown in the patient information table 51 and the examination information shown in the examination information table 52. The patient information-examination information table 53 associates one piece of patient information with one or more pieces of examination information.

An observation table 54 stores observation data about the observation of a doctor on an examination result, and a observation table 54 is generated for each examination item of a performed examination.

A treatment table 55 stores treatment data indicating the contents of the treatment performed corresponding to an examination, and one treatment table 55 is generated for each treatment executed on a performed examination.

An observation-treatment table 56 shows the relationship between the observation table 54 and the treatment table 55. The observation-treatment table 56 associates a piece of examination information in the examination information table 52 with one or more observation tables 54, and each observation table 54 is associated with one or more treatment tables 55.

A diagnosis table 57 stores diagnosis data indicating a result diagnosed by a doctor based on the observation on an examination result and a result of a treatment after an examination. The diagnosis table 57 is generated according to the above-mentioned examination information, and stores diagnosis data for each diagnosis item.

A comment table 58 stores comment data about a comment generated as necessary on the entire examination result. The comment table 58 is generated according to the above-mentioned examination information, and stores various comments on a diagnosis report, a pathological order, an instruction after an examination, etc.

Described below are the details of each of the processes of generating, editing, and recording a diagnosis report performed by the system shown in FIG. 2A. Each of the processes described below is performed by the CPUs 41 of the server device 10 and the terminal devices 20-1, 20-2, and 20-3. The CPU 41 of the server device 10 executes the above-mentioned control program stored in advance in the storage unit 44 of the server device 10, and the CPUs 41 of the terminal devices 20-1, 20-2, and 20-3 execute the above-mentioned control programs stored in advance in the storage units 44 of the terminal devices 20-1, 20-2, and 20-3. Thus, the processes are realized by the CPUs 41 of the respective devices.

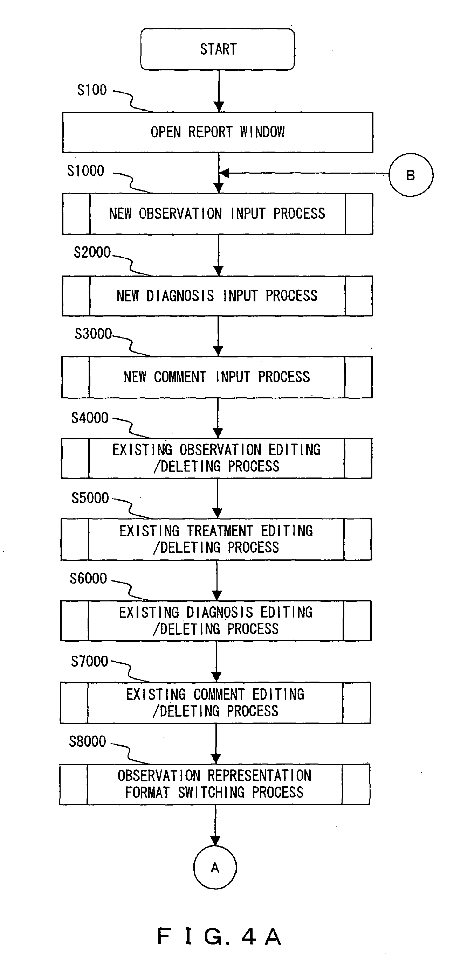

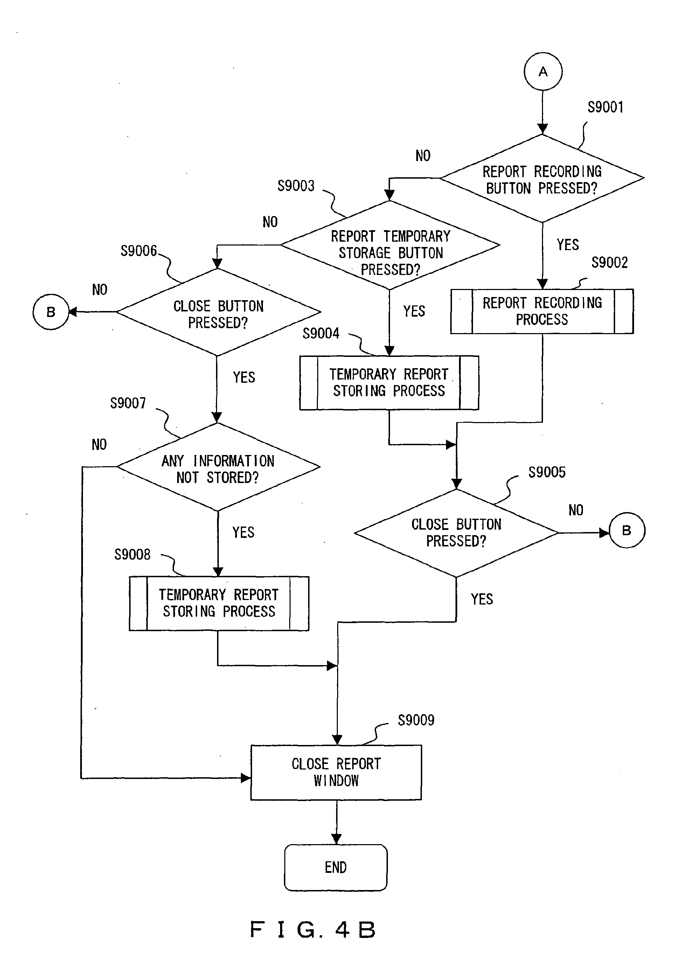

First described are the flowcharts in FIGS. 4A and 4B. These flowcharts indicate the contents of the processes of the report window process. The processes are started when the CPU 41 receives an instruction issued by an operation on the input units 45 of the terminal devices 20-1, 20-2, and 20-3 to generate, edit, and record a diagnosis report of a performed examination.

In FIG. 4A, the display unit 46 of the terminal devices 20-1, 20-2, or 20-3 displays the report window screen in S100.

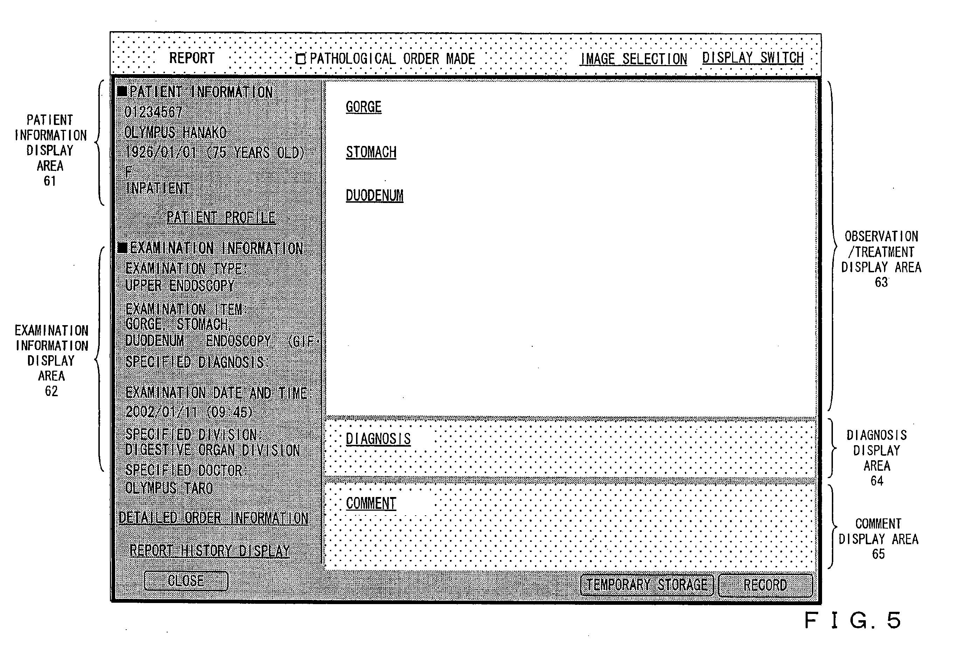

FIG. 5 shows an example of a report window screen. The report window screen shown in FIG. 5 has a patient information display area 61, an examination information display area 62, an observation/treatment display area 63, a diagnosis display area 64, and a comment display area 65.

The above-mentioned patient information is displayed in the patient information display area 61, and the above-mentioned examination information is displayed in the examination information display area 62. They are displayed according to the patient information and the examination information whose relationship is shown in the patient information-examination information table 53 in the database of the server device 10. The patient information and the examination information are read and transmitted from the patient information table 51 and the examination information table 52 respectively.

The contents of the observation indicated by the above-mentioned observation data are displayed by examination item, and the contents of the treatment corresponding to the displayed observation are displayed in the observation/treatment display area 63. In the example shown in FIG. 5, since the contents of the observation and the treatment have not been input yet, only the title of an examination item is displayed with an underline in the observation/treatment display area 63.

The contents of the diagnosis displayed by the above-mentioned diagnosis data are displayed in the diagnosis display area 64, and the contents of the comment displayed by the above-mentioned comment data are displayed in the comment display area 65. In the display shown in FIG. 5, since the contents of the diagnosis and the comment have not been input yet, only the character strings of the "diagnosis" and the "comment" are displayed with the underlines respectively in the diagnosis display area 64 and the observation table 54.

Described below is the explanation by referring to FIG. 4A again.

When the process in S100 is completed, the new observation input process in S1000 is performed. Subsequently, the new diagnosis input process in S2000, the new comment input process in S3000, the existing observation editing/deleting process in S4000, the existing treatment editing/deleting process in S5000, the existing diagnosis editing/deleting process in S6000, the existing comment editing/deleting process in S7000, and the observation representation format switching process in S8000 are performed in this order.

In S9001 shown in FIG. 4B after the process in S8000 shown in FIG. 4A, it is determined whether or not a report recording button (a "record" button displayed at the lower right portion on the screen shown in FIG. 5) has been pressed. If the determination result is YES, control is passed to S9002. If the determination result is NO, control is passed to S9003.

In the explanation of the present embodiment, "pressing a button" refers to a clicking operation, etc. with the mouse in the position of a pointer which is moved to the position on the screen as indicated by an arrow, etc. superposed on the screen of the display unit 46 by operating the mouse, etc. of the input unit 45 by the operator of the terminal devices 20-1, 20-2, and 20-3.

The report recording process is performed in S9002. In this process, a diagnosis report generated and edited in the report window process is stored in the database of the server device 10. To be more concrete, the process includes the steps of: transmitting to the server device 10 the observation data, the treatment data, the diagnosis data, and the comment data which form the diagnosis report on a performed examination; generating and storing by the server device 10 the observation table 54 and the treatment table 55 of the observation data and the treatment data respectively; and allowing the server device 10 to perform the process of showing the correspondence between the observation data and the treatment data on the observation-treatment table 56 and also allowing the server device 10 to perform the process of generating and storing the diagnosis data and the comment data in the diagnosis table 57 and the treatment table 55 respectively. After the process in S9002 is completed, control is passed to S9005.

In S9003, it is determined whether or not the report temporary storage button (a "temporary storage" button to the left of the report recording button at the lower right portion on the screen shown in FIG. 5) has been pressed. If the determination result is YES, control is passed to S9004. If the determination result is NO, control is passed to S9006.

In S9004, the temporary report storing process is performed. In this process, the diagnosis report generated and edited in the report window process is not stored in the database of the server device 10, but is temporarily stored in the storage units 44 of the terminal devices 20-1, 20-2, and 20-3. Practically, in this process, the observation data, the treatment data, the diagnosis data, and the comment data forming a diagnosis report on a performed examination are stored in the storage unit 44 together with the patient information and the examination information. If the above-mentioned process in S100 is performed when the storage units 44 of the terminal devices 20-1, 20-2, and 20-3 store the data, the report window screen showing these data is displayed on the display unit 46.

In S9005, it is determined whether or not the "close" button (at the lower left portion on the screen shown in FIG. 5) has been pressed. If the determination result is YES, control is passed to S9009. If the determination result is NO, then control is returned to S1000 shown in FIG. 4A, and the above-mentioned process is repeated.

In S9006, as in the process in S9005, it is determined whether or not the "close" button has been pressed. If the determination result is YES, control is passed to S9007. If the determination result is NO, control is returned to step S1000, and the above-mentioned process is repeated.

In S9007, it is determined whether or not any of the observation data, the treatment data, the diagnosis data, and the comment data forming a diagnosis report on a performed examination is not to be stored because the report recording process in S9002 or the temporary report storing process in S9004 has not been performed after any of the data has been generated and updated. If the determination result is YES, the temporary report storing process similar to the process in S9004 is performed in S9008.

In S9009, the process of closing the report window screen displayed on the display unit 46 is performed. Afterwards, the report window process terminates.

The above-mentioned process is the report window process, and a diagnosis report is generated, edited, and recorded by performing the process in the system shown in FIG. 2A.

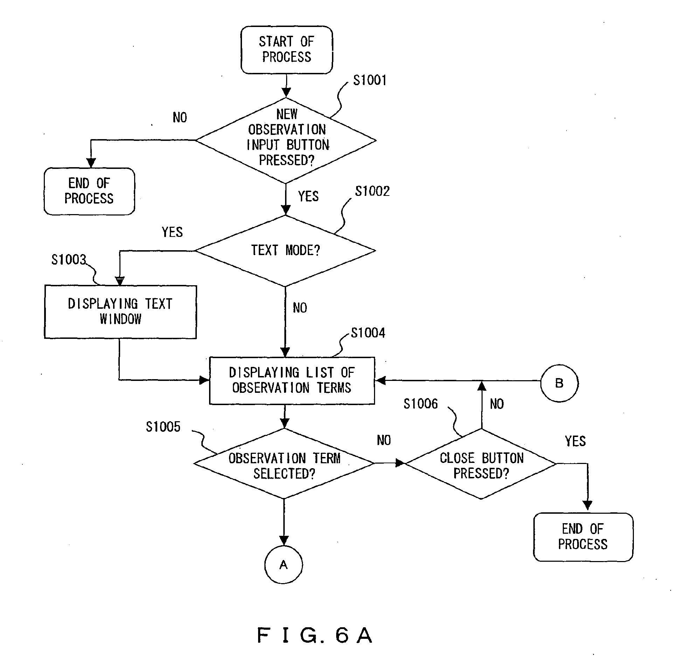

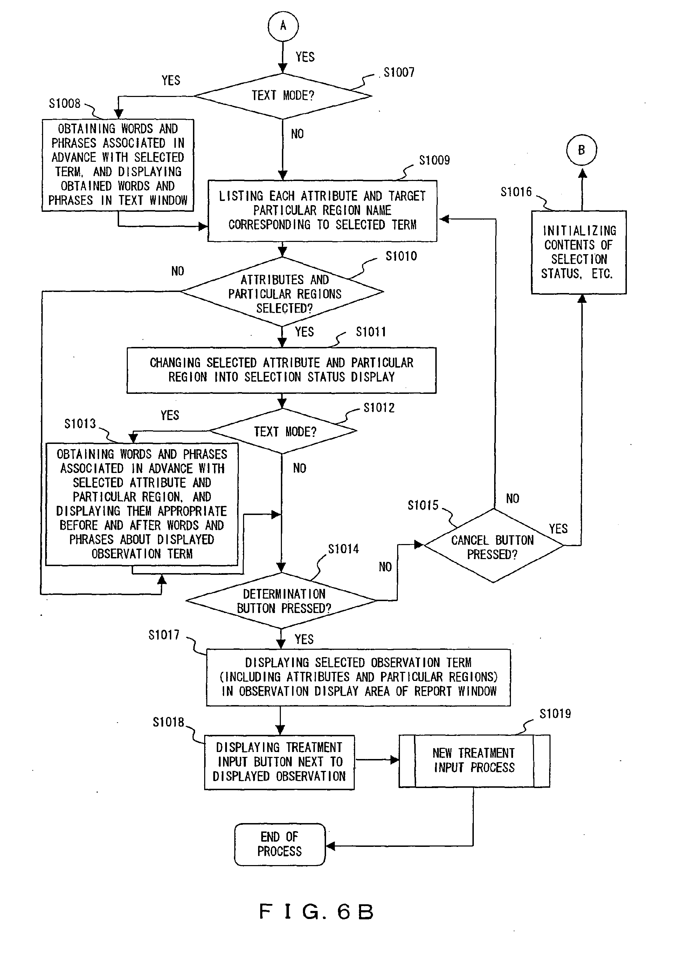

Described below are the flowcharts shown in FIGS. 6A and 6B. FIGS. 6A and 6B are flowcharts of the processes of the new observation input process in S1000 shown in FIG. 4A. The process is performed to newly input observation in the diagnosis report.

First, in S1001 shown in FIG. 6A, it is determined whether or not the new observation input button has been pressed. If the determination result is YES, control is passed to S1002. If the determination result is NO, then the new observation input process terminates, and control is returned to the process shown in FIG. 4A.

The new observation input button refers to the a character string indicating the title of an examination item such as "gorge", "stomach", "duodenum", etc. displayed with underlines in the observation/treatment display area 63 on the report window screen shown in FIG. 5.

In S1002, it is determined whether or not the representation format mode which can be switched in the representation format switching process described later is set in the "text mode". If the determination result is YES, the text window is superposed on the report window screen displayed on the display unit 46. The text window is described later.

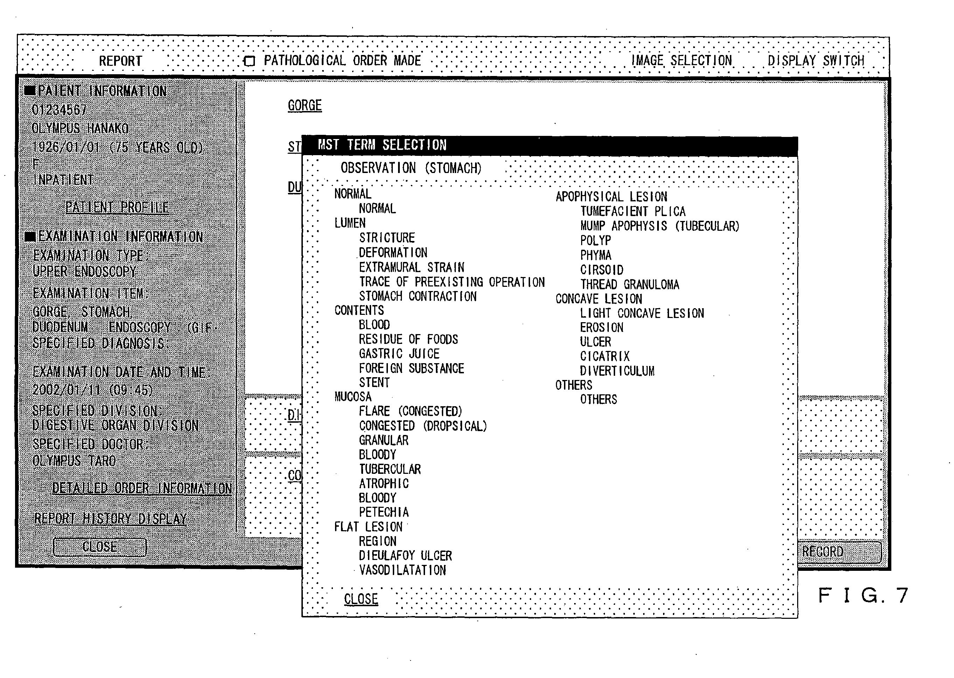

In S1004, a list of observation terms can be superposed on the report window screen displayed on the display unit 46. FIG. 7 shows a display example. The display example shown in FIG. 7 is a list of observation terms about the stomach displayed when the character string of the "stomach" in the observation/treatment display area 63 is pressed in the new observation input buttons.

In S1005, it is determined whether or not a term has been selected from the list of observation terms. If the determination result is YES, control is passed to S1007 shown in FIG. 6B. If the determination result is NO, control is passed to S1006.

In S1006, it is determined whether or not the "close" button at the lower right of the list of the observation term shown in FIG. 7 has been pressed. If the determination result is YES, the new observation input process terminates as is, and control is returned to the process shown in FIG. 4A. If the determination result is NO, control is returned to the process in S1004, and the above-mentioned processes are repeated.

Back in FIG. 6B, it is determined in S1007 whether or not the representation format mode described later has been set in the "text mode". If the determination result is YES, a word or a phrase (a part of a word or a combination of a derivative of a word, a preposition, an auxiliary word, etc.) associated in advance with an observation term selected from the list is obtained, and the obtained word and phrase are displayed in the text window in S1008.

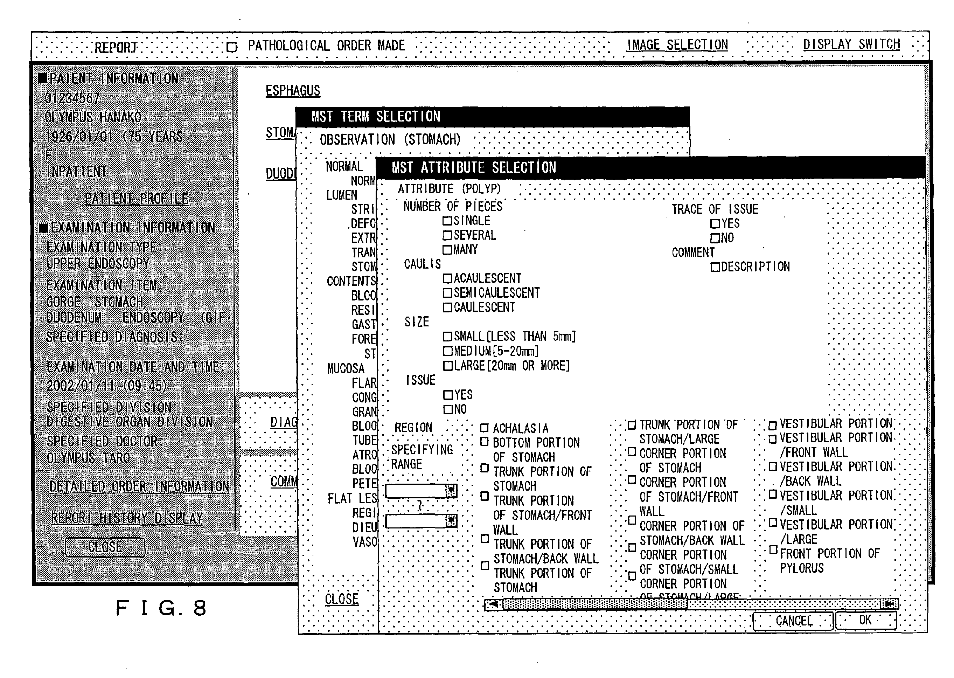

In S1009, a list of various attributes and target particular regions corresponding to the observation terms selected from the list is superposed on the display unit 46. FIG. 8 shows a display example. The display example shown in FIG. 8 shows an example of the list of the attributes and target particular regions about the "polyp" selected from the list of observation terms about the stomach. The list is displayed by selecting and pressing the character string of the "polyp" in the list of the observation terms about the stomach.

In S1010, it is determined whether or not the attribute and the target particular region have been selected from the list shown in FIG. 8. If the determination result is YES, control is passed to S1011. If the determination result is NO, control is passed to S1014.

In S1011, the display of the attribute and target particular region selected in the preceding step is changed into the selection status display. If it is the example shown in FIG. 8, a check mark is given to the check box corresponding to the display of the selected attribute and target particular region.

In S1012, it is determined whether or not the representation format mode described later has been set in the "text mode". If the determination result is YES, the word and phrase associated in advance with the attribute and target particular region selected from the list are obtained, and the obtained word and phrase are appropriately arranged before and after the word and phrase corresponding to the observation terms displayed in the text window, thereby generating the text.

In S1014, it is determined whether or not a determination button (an "OK" button provided at the lower right portion in the list of the attributes and target particular regions shown in FIG. 8) has been pressed. If the determination result is YES, control is passed to S1017. If the determination result is NO, control is passed to S1015.

In S1015, it is determined whether or not a cancel button (a "cancel" button displayed to the left of the determination button at the lower right portion on the screen shown in FIG. 8) has been pressed. If the determination result is YES, control is passed to S1016. If the determination result is NO, control is returned to S1009, thereby repeating the above-mentioned processes.

In S1016, the selected contents of the attributes and target particular regions detected in each of the preceding steps are initialized, and control is returned to a no selection status. Afterwards, control is returned to S1004, thereby repeating the above-mentioned processes.

In S1017, the selected observation terms, attributes, and the target particular regions in the preceding processes are displayed in the observation/treatment display area 63 on the report window screen. The displayed items in this process are the contents of the generated observation.

Then, in S1018, a treatment input button is displayed to the right of the observation displayed in the observation/treatment display area 63.

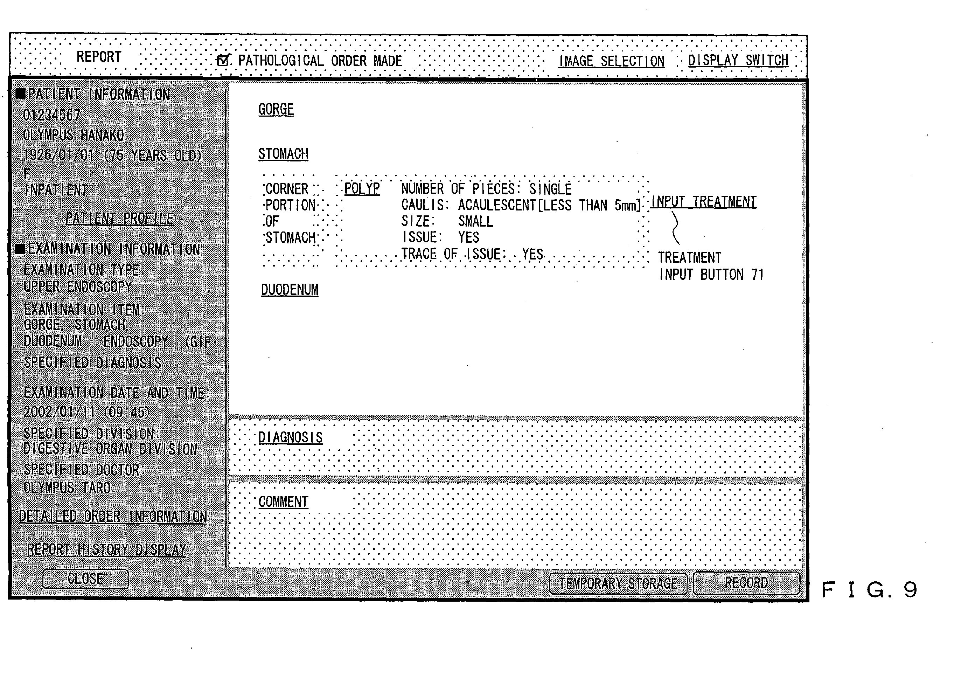

FIG. 9 shows an example of an observation displayed on the display unit 46 in the processes in S1017 and S1018. In FIG. 9, the display of a character string of the underlined "input treatment" in FIG. 9 refers to a treatment input button 71 displayed in the process in S1018. By newly displaying the treatment input button 71, the system user can be prompted to input the treatment corresponding to the input observation.

In S1019, the new treatment input process is performed. In this process, the treatment for the observation generated in the preceding processes can be newly input. Thus, a treatment inputting operation as an operation following the observation inputting operation is selected by priority is an important point in the present invention. This reflects the standard procedure of generating a diagnosis report.

After completing the process in S1019, the new observation input process is terminated, and control is returned to the process shown in FIG. 4A.

The above-mentioned process is the new observation input process.

As described above, in the system shown in FIG. 2A, the observation is generated by selecting an observation term from a prepared list of observation terms and by selecting an attribute and a target particular region corresponding to the selected observation term from a list of various attributes and target particular regions. Therefore, the words and phrases used in the observation can be standardized regardless of the doctors. As a result, any doctor referring to the generated observation can easily understand the contents of the observation, and the information in the observation can be widely used, thereby easily generating a database for secondary use of the information.

Described below is the text window in the above-mentioned new observation input process.

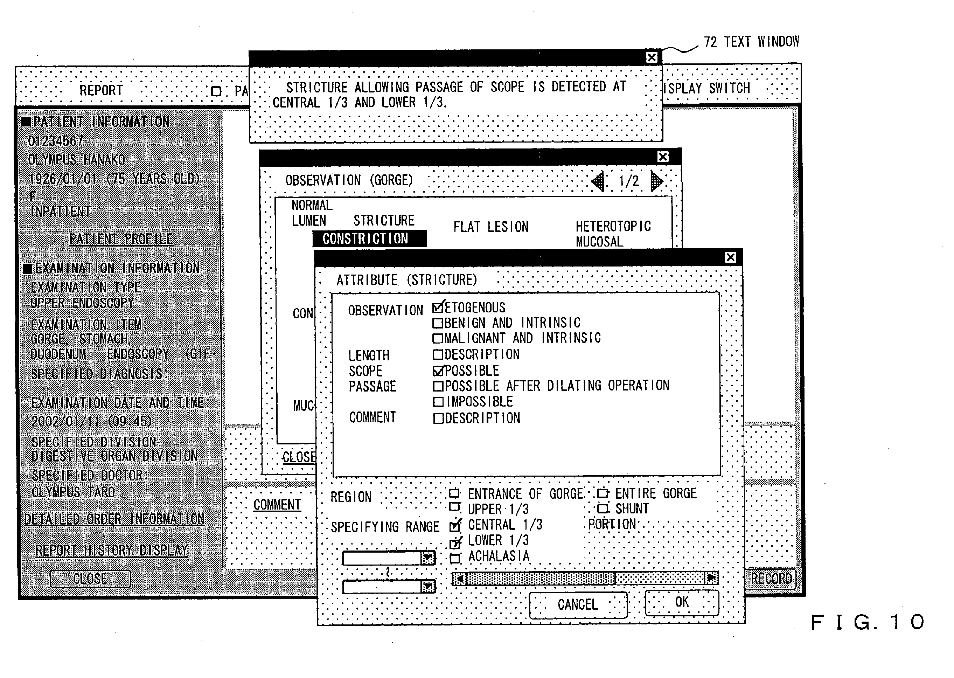

FIG. 10 shows a display example of a list of attributes and target particular regions corresponding to the observation term in the text mode, and an example of a screen displayed on the display unit 46 in the process in S1013 shown in FIG. 6B. When the above-mentioned representation format mode is set in a text mode, the word and phrase associated in advance with the attribute and target particular region selected from the list are obtained, and the text formed by arranging the obtained word and phrase before and after the word and phrase associated with the terms in the observation generated in the process in S1008 shown in FIG. 6B is displayed in a text window 72, as shown in FIG. 10.

If it is detected in the determining process in S1014 that the determination button has been pressed, the text displayed in the text window 72, that is, the text indicating the contents of the observation, is displayed in the observation/treatment display area 63 of the report window screen in the process in S1017, and the treatment input button is displayed on the right of the observation text in the process in S1018.

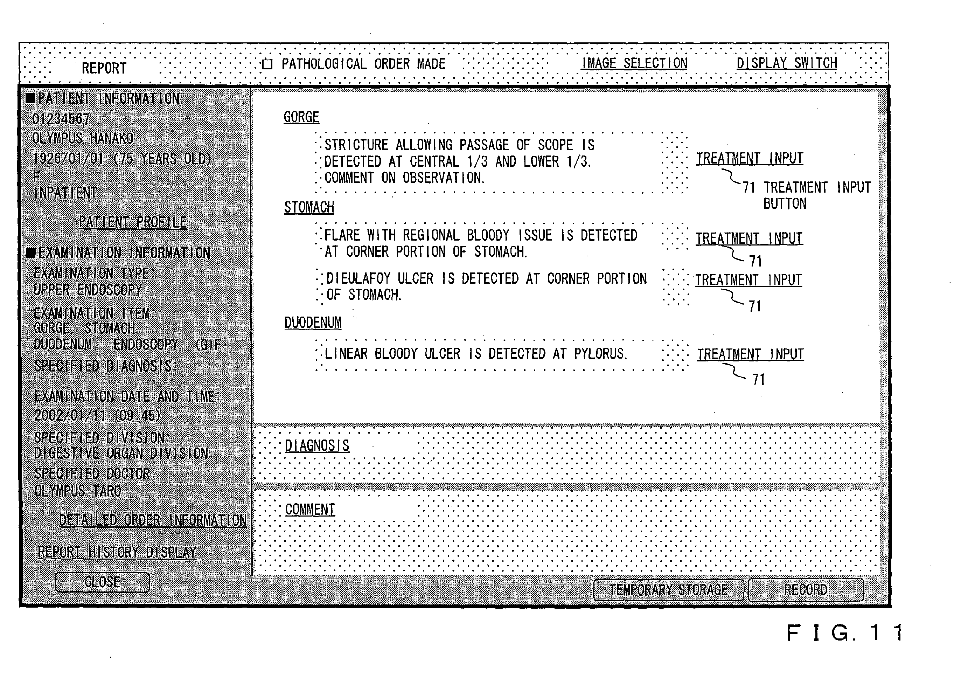

FIG. 11 shows a display example of observation displayed on the display unit 46 in the above-mentioned process. The display of the underlined character string "input treatment" in FIG. 11 is the treatment input button 71 displayed in the process in S1018.

As clearly indicated by the display example shown in FIG. 11 in comparison with the display example shown in FIG. 9, the contents of the observation generated in the text mode are displayed not as a list of words and phrases, but as text. Therefore, it is helpful for a patient, etc. having little medical knowledge to understand the contents of the observation.

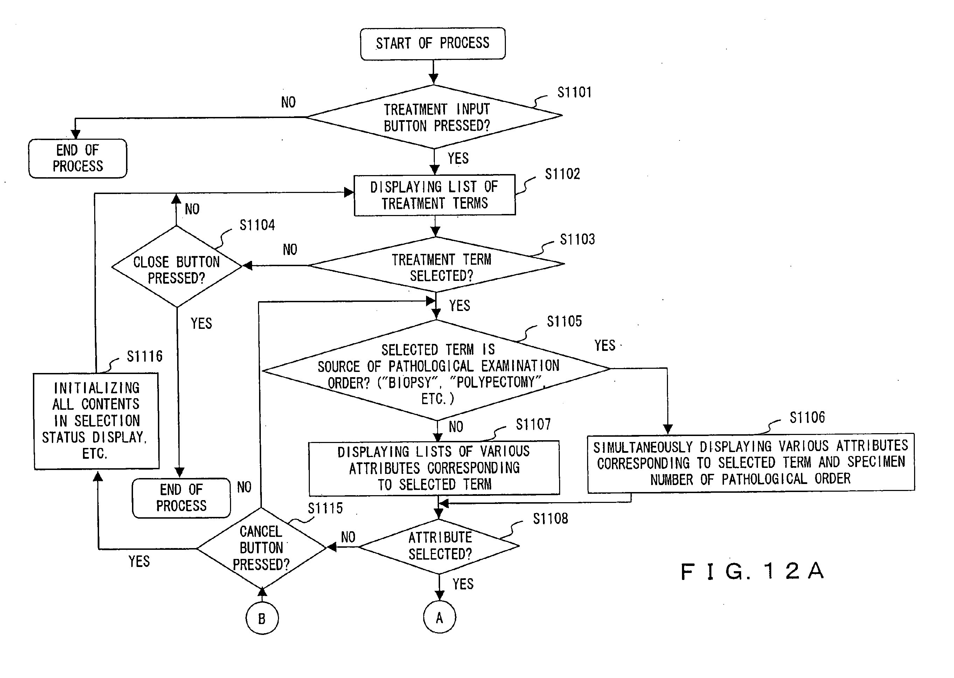

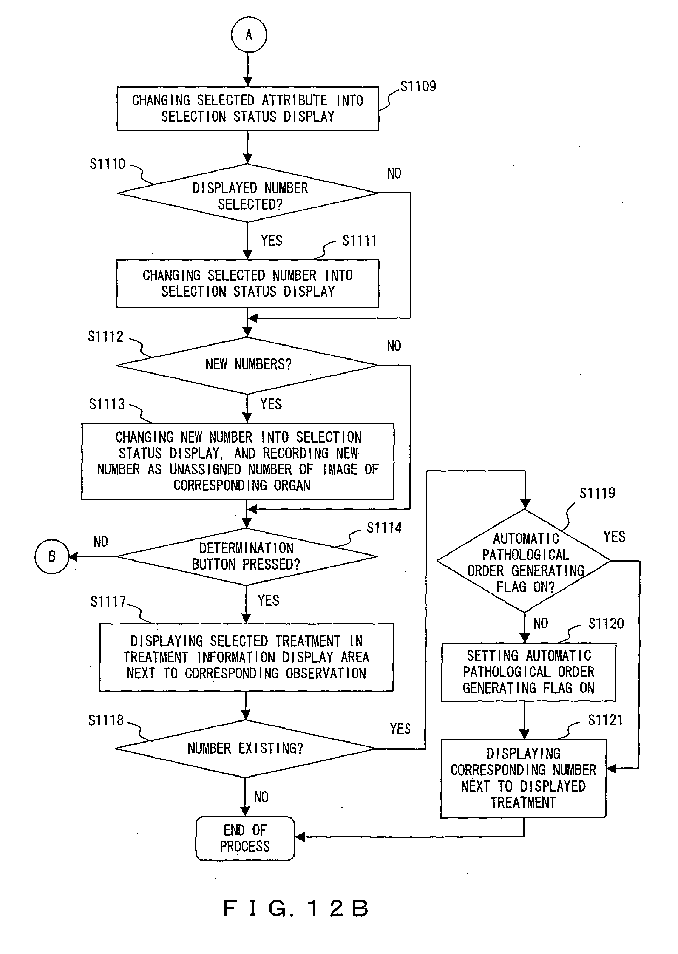

Described below are the flowcharts shown in FIGS. 12A and 12B. FIGS. 12A and 12B are the flowcharts indicating the contents of the new treatment input process in S1019 shown in FIG. 6B. In this process, a new input is made for the treatment corresponding to the observation in the diagnosis report.

In this explanation, it is assumed that the word and phrase indicating the contents of the observation and the treatment input button 71 are displayed in the observation/treatment display area 63. That is, it is assumed that the new treatment input process is started with the report window screen shown in FIG. 9 displayed on the display units 46 of the terminal devices 20-1, 20-2, and 20-3.

In S1101 shown in FIG. 12A, it is determined whether or not the treatment input button 71 displayed in the observation/treatment display area 63 has been pressed. If the determination result is YES, control is passed to the process in S1102. If the determination result is NO, the new treatment input process is terminated as is, and control is returned to the process shown in FIG. 6B.

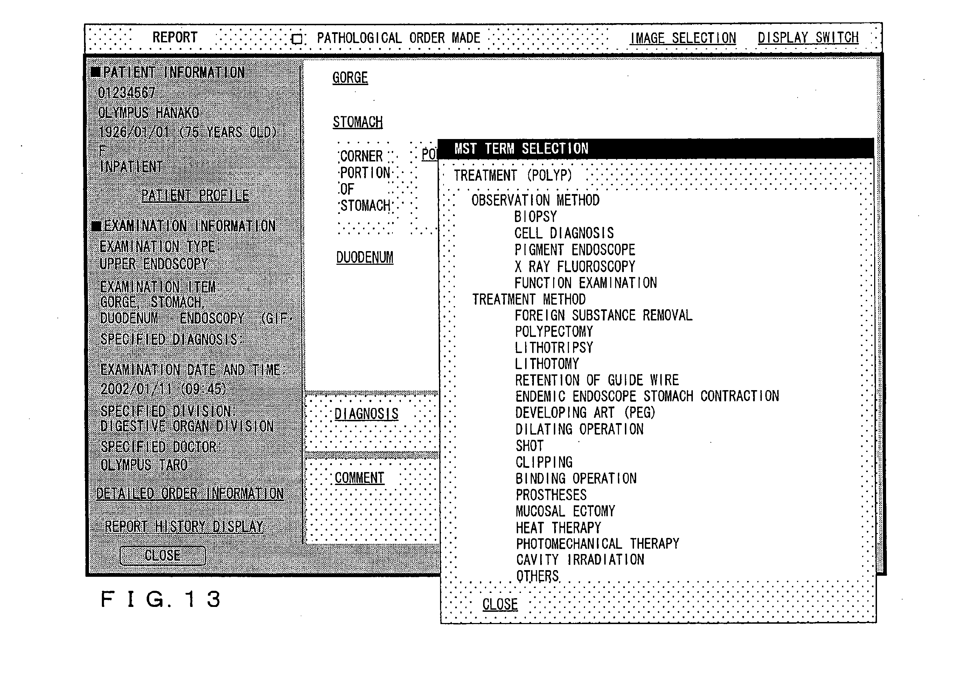

In S1102, a list of treatment terms is superposed on the report window screen displayed on the display unit 46. FIG. 13 shows a display example. The display example shown in FIG. 13 is a list of treatment terms about the stomach (polyp), and the list is displayed when it is detected that the treatment input button 71 displayed on the right of the contents of the observation about the stomach in the observation/treatment display area 63 has been pressed.

In S1103, it is determined whether or not a selection has been detected from the list of the treatment terms. If the determination result is YES, control is passed to S1105. If the determination result is NO, control is passed to S1104.

In S1104, it is determined whether or not the "close" button at the lower left portion of the list of the treatment terms shown in FIG. 13 has been pressed. If the determination result is YES, the new treatment input process is terminated as is, and control is returned to the process in FIG. 6B. If the determination result is NO, then control is returned to the process in S1102, and the above-mentioned processes are repeated.

In S1105, it is determined whether or not the treatment term selected from the list is predetermined in the pathological examination request (pathological examination order) (for example, "biopsy", "polypectomy", etc.). If the determination result is YES, control is passed to S1106. If the determination result is NO, control is passed to S1107.

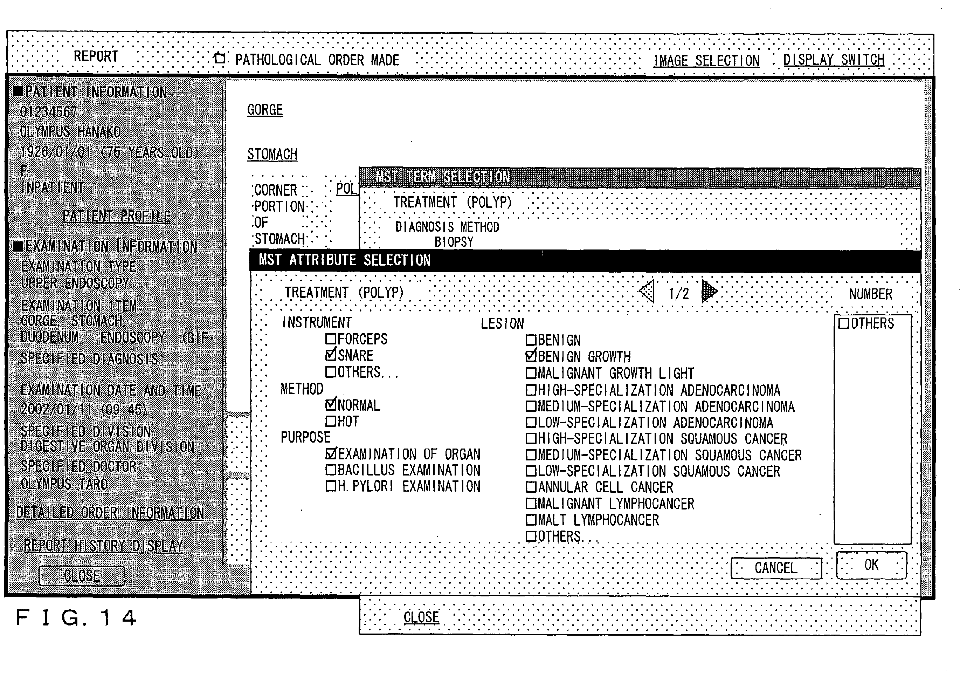

In S1106, a window screen having a list of various attributes corresponding to the treatment term selected from the list and an input column of the number (specimen number) assigned to the specimen as a target of the pathological order is superposed on the display unit 46. FIG. 14 shows a display example. The display example shown in FIG. 14 is an example of a window screen having a list of attributes about the "biopsy" selected from the list of the treatment terms about the stomach and an input column of a specimen number. The screen is displayed when the character string "biopsy" in the list of the treatment terms about the stomach shown in FIG. 13 is pressed and selected.

After the process in S1106, control is passed to the process in S1108.

In S1107, a list of various attributes corresponding to the treatment term selected from the list is superposed on the display unit 46. The screen of the list is displayed with the input column of a specimen number removed from the window screen.

In S1108, it is determined whether or not an attribute has been selected from the list displayed in the process in S1106 or S1107. If the determination result is YES, control is passed to S1109 shown in FIG. 12B. If the determination result is NO, control is passed to the process in S1115.

Now in FIG. 12B, the display of the attribute selected and detected in the process of the preceding step is changed into the selection status display in S1109. In the example shown in FIG. 14, a check mark is given to the check box corresponding to the display of the selected and detected attribute.

In S1110, it is determined whether or not a number displayed in the input column of the specimen number of the window screen displayed on the display unit 46 has been selected. If the determination result is YES, the display is changed into a display indicating the selected number in S1111.

In the screen example shown in FIG. 14, the status in which no specimen number has been selected is shown. Therefore, the input column of the specimen number shows only the display of the character string "others".

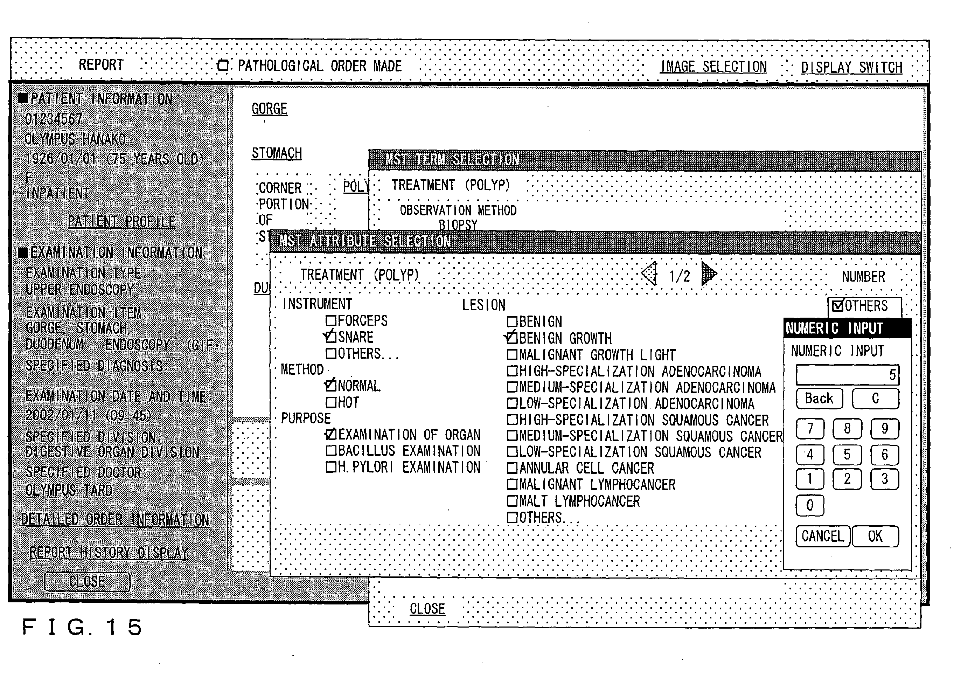

In S1112, it is determined whether or not a new number has been selected. In the screen example shown in FIG. 14, the numeric input screen shown in FIG. 15 is displayed depending on the selection of the display of the character string "others", and it is determined whether or not a numerial has been input by pressing the ten key buttons provided for the screen. If the determination result is YES, the display of the number is changed into the display indicating that the numeric display is a specimen number in S1113. If there is the image data of the organ from which the specimen has been obtained, the specimen number is displayed in the position of the specimen in the image of the organ.

A new number can be automatically obtained by the terminal devices 20-1, 20-2, and 20-3.

In S1114, it is determined whether or not the determination button (the "OK" button at the lower right portion on the screen shown in FIG. 14) has been pressed. If the determination result is YES, control is passed to S1117. If the determination result is NO, control is passed to S1115 shown in FIG. 12A.

Back in FIG. 12A, in S1115, it is determined whether or not the cancel button (the "cancel" button shown at the lower right portion of the screen shown in FIG. 14) has been pressed. If the determination result is YES, control is passed to S1116. If the determination result is NO, control is returned to S1105, and the above-mentioned processes are repeated.

In S1116, the selection contents of the attributes detected in the process of each step are initialized, and control is returned to the no selection status. Afterwards, control is returned to S1102, and the above-mentioned processes are repeated.

On the other hand, in S1117 shown in FIG. 12B, the selected treatment term and the attribute in the preceding processes are displayed in the display area of the treatment information in the observation/treatment display area 63 of the report window screen on the right of the treatment input button 71 whose press is detected in S1101. The displayed data in this process is the contents of the treatment generated corresponding to the contents of the observation. In the observation/treatment display area 63, the contents of the corresponding observation and the contents of the treatment are displayed in parallel for each examination item.

In S1118, it is determined whether or not a specimen number has been selected. If the determination result is YES, control is passed to S1119. If the determination result is NO, the new treatment input process is terminated, and control is returned to the process shown in FIG. 6B.

In S1119, it is determined whether or not the flag (automatic pathological order generating flag) indicating whether or not a pathological order is to be automatically generated is set ON. If the determination result is NO, the automatic pathological order generating flag is set ON in S1120.

In S1121, the selected specimen number is displayed in the position to the right of the word and phrase relating to the treatment and displayed in the observation/treatment display area 63 of the report window screen in the above-mentioned process in S1117.

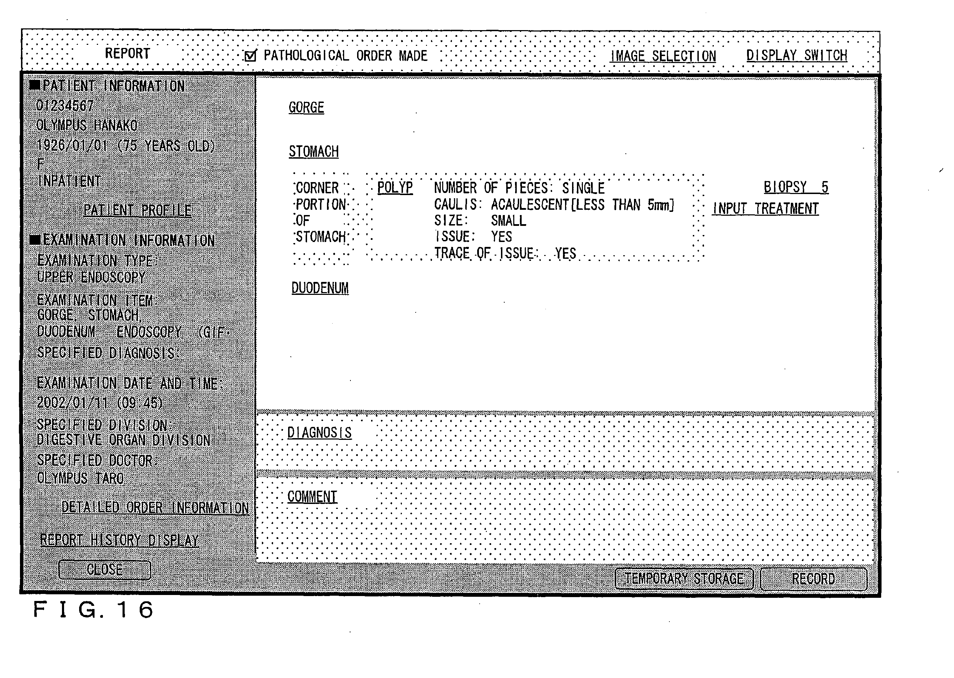

FIG. 16 shows a display example of the observation displayed on the display unit 46 in the processes in S1117 and S1121. In the display of the character string "biopsy 5" in the display area of the treatment information in the observation/treatment display area 63 shown in FIG. 16, "biopsy" is displayed in the process in S1117, and "5" is displayed in the process in S1121.

After the process in S1121, the new treatment input process is terminated, and control is returned to the process shown in FIG. 6B.

The above-mentioned processes are the new treatment input process.

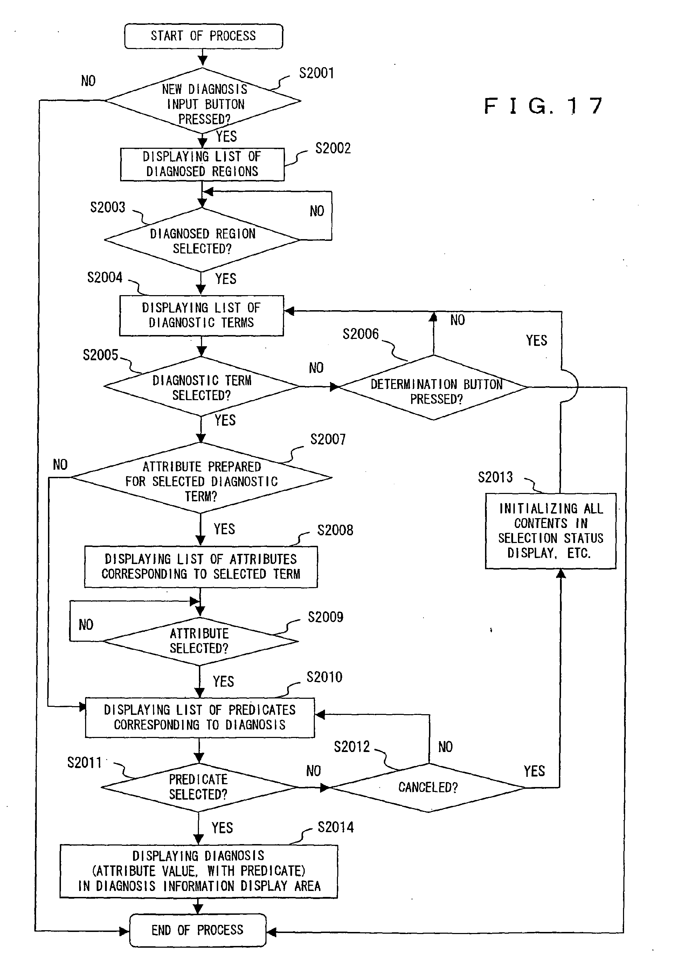

Described below are the processes shown in FIG. 17. FIG. 17 is a flowchart of the contents of the new diagnosis input process in S2000 shown in FIG. 4A. This process is performed for newly inputting a diagnosis based on the result of observation and treatment.

In the explanation, it is assumed that the new diagnosis input process is started with the report window screen on which data has been input to the observation/treatment display area 63 as shown in FIG. 16 displayed in the display units 46 of the terminal devices 20-1, 20-2, and 20-3.

First in S2001, it is determined whether or not a new diagnosis input button has been pressed. If the determination result is YES, control is passed to S2002. If the determination result is NO, the new diagnosis input process is terminated as is, and control is returned to the process shown in FIG. 4A. The new diagnosis input button refers to the character string "diagnosis" displayed with an underline in the diagnosis display area 64 (FIG. 5) of the report window screen.

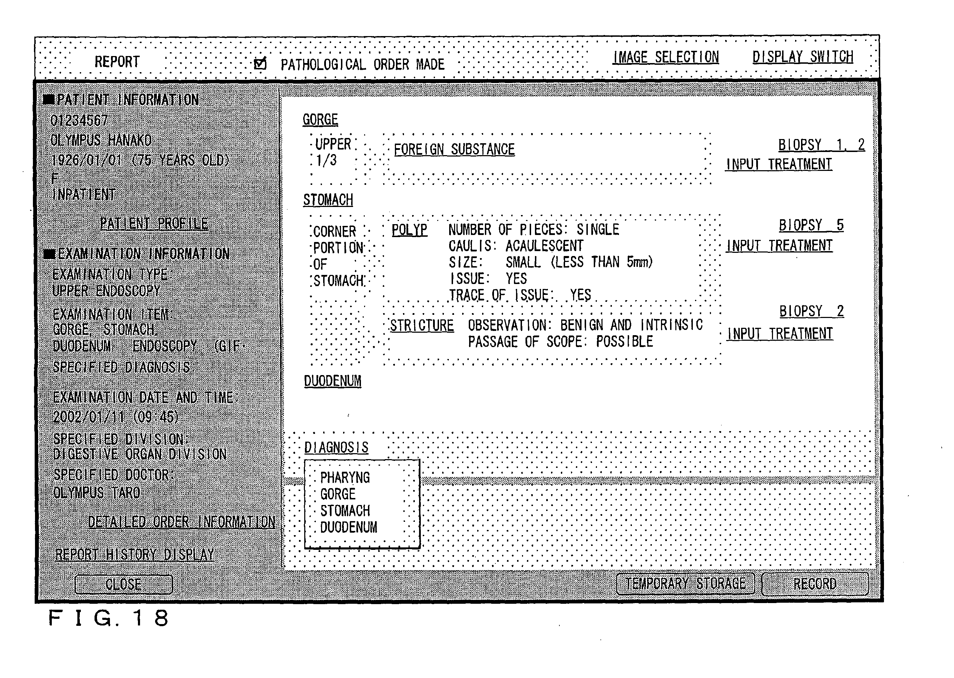

In S2002, a list of diagnosed regions are superposed on the report window screen displayed on the display unit 46. FIG. 18 shows a display example. The display example shown in FIG. 18 is generated based on the title of an examination item displayed in the observation/treatment display area 63.

In S2003, it is determined whether or not a diagnosed region has been selected from a list of the diagnosed regions. The process in S2003 is repeated until the determination result is YES.

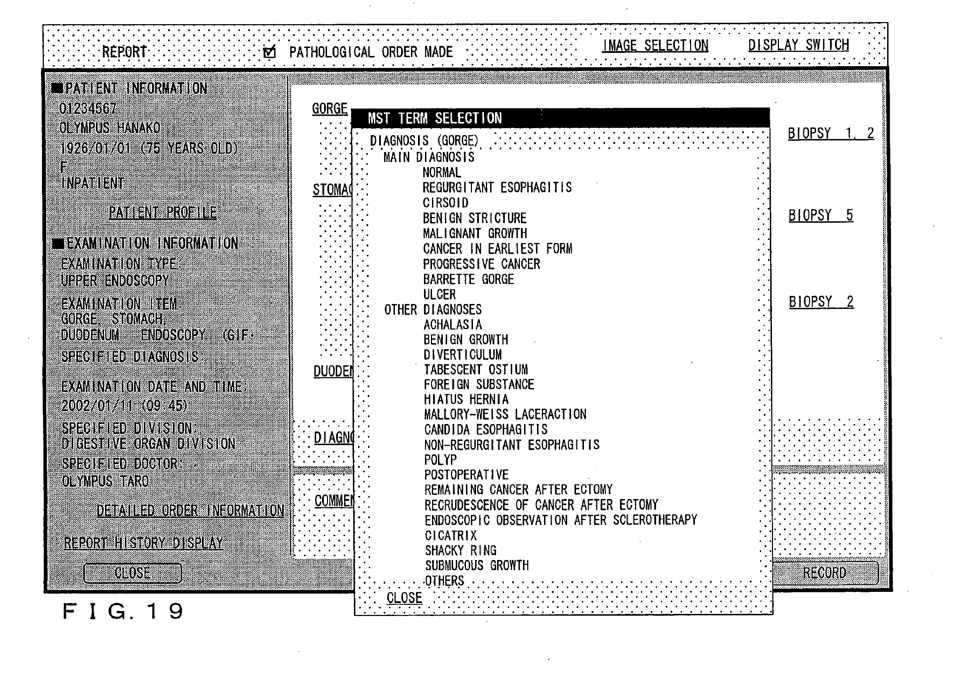

In S2004, a list of diagnostic terms is superposed on the report window screen displayed on the display unit 46. FIG. 19 shows a display example. The display example shown in FIG. 19 shows a list of diagnostic terms about "gorge". The list is prepared corresponding to each diagnosed region, and a list corresponding to the diagnosed region detected in the process in S2003 is displayed.

In S2005, it is determined whether or not a diagnostic term has been selected from a list of diagnosed regions. If the determination result is YES, control is passed to S2007. If the determination result is NO, control is passed to S2006.

In S2006, it is determined whether or not the "close" button provided at the lower left portion of the list of the diagnostic terms shown in FIG. 19 has been pressed. If the determination result is YES, the new diagnosis input process is terminated as is, and control is returned to the process shown in FIG. 4A. If the determination result is NO, control is returned to S2004, and the above-mentioned processes are repeated.

In S2007, it is determined whether or not a list of various attributes corresponding to the diagnostic term selected from the list has been prepared. If the determination result is YES, control is passed to S2008. If the determination result is NO, control is passed to S2010.

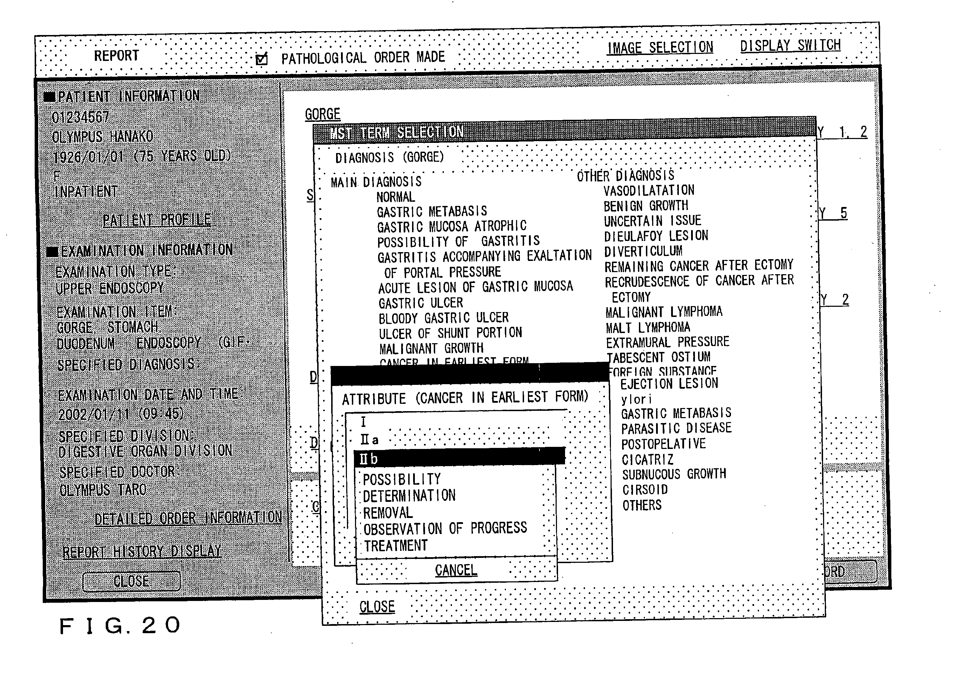

In S2008, a list of various attributes corresponding to a diagnostic term selected from the list is superposed on the display unit 46. FIG. 20 shows a display example. The display example shown in FIG. 20 is displayed when "cancer in earliest form" is selected as a diagnostic term about the "stomach", and is displayed after the superposed display process in S2010 described later is displayed.

In S2009, it is determined whether or not an attribute has been selected from the displayed. The process in S2009 is repeated until the determination result is YES.

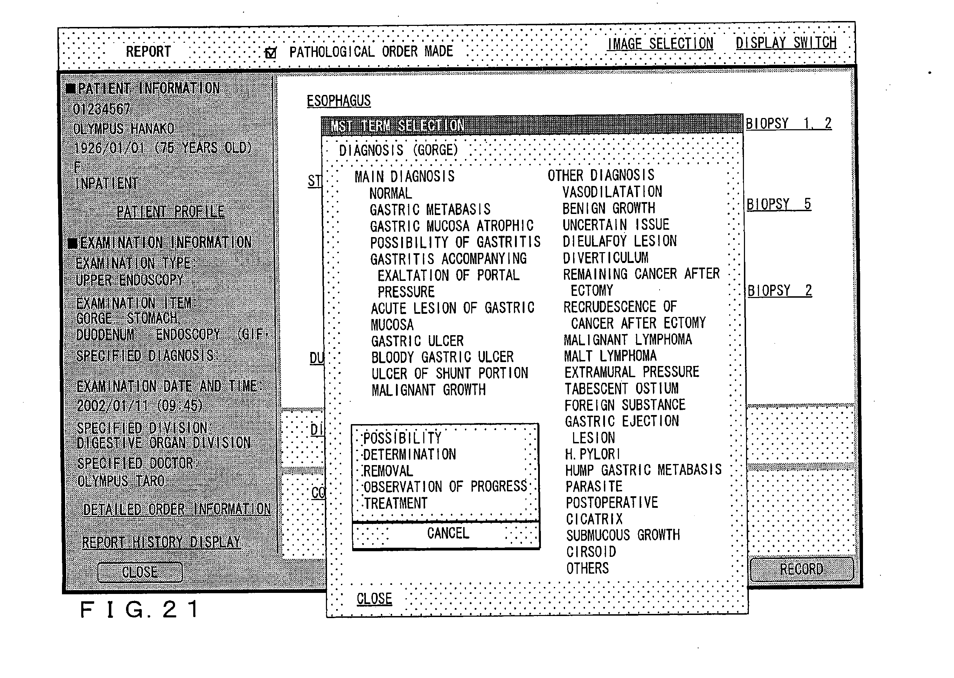

In S2010, a list of predicates corresponding to a diagnostic term selected from the list is superposed on the display unit 46. FIG. 21 shows a display example. The display example shown in FIG. 21 is displayed when "malignant growth" is selected as a diagnostic term. The display example shown in FIG. 20 is also displayed on the display unit 46 in the process in S2010.

In S2011, it is determined whether or not a predicate has been selected from the displayed list. If the determination result is YES, control is passed to S2014. If the determination result is NO, control is passed to S2012.

In S2012, it is determined whether or not a character string "cancel" in the bottom line of the displayed list of the predicates has been selected. If the determination result is YES, control is passed to S2013. If the determination result is NO, control is returned to S2010, and the above-mentioned processes are repeated.

In S2013, the selected contents of the attributes detected in the process in each step are initialized, and control is returned to the non selection status. Afterwards, control is returned to S2004, and the above-mentioned processes are repeated.

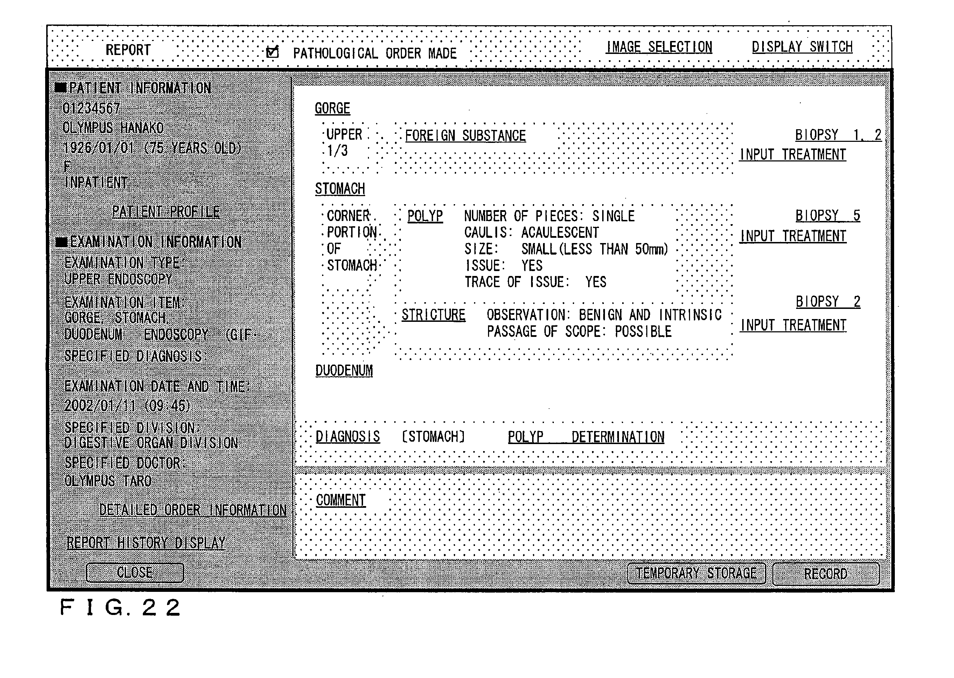

In S2014, the diagnostic terms, the attributes, and the predicates selected in the preceding processes are associated with the titles of examination items, and displayed in the diagnosis display area 64 of the report window screen. FIG. 22 shows a display example. The diagnosis display area 64 shown in FIG. 22 displays the contents of the diagnosis about the "stomach".

The above-mentioned processes are the new diagnosis input process.

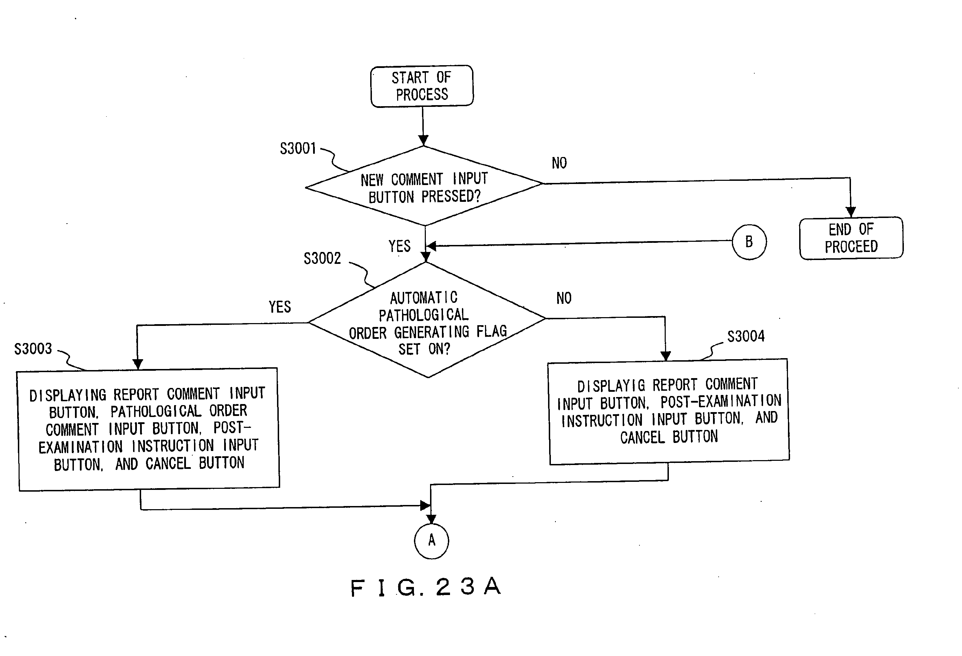

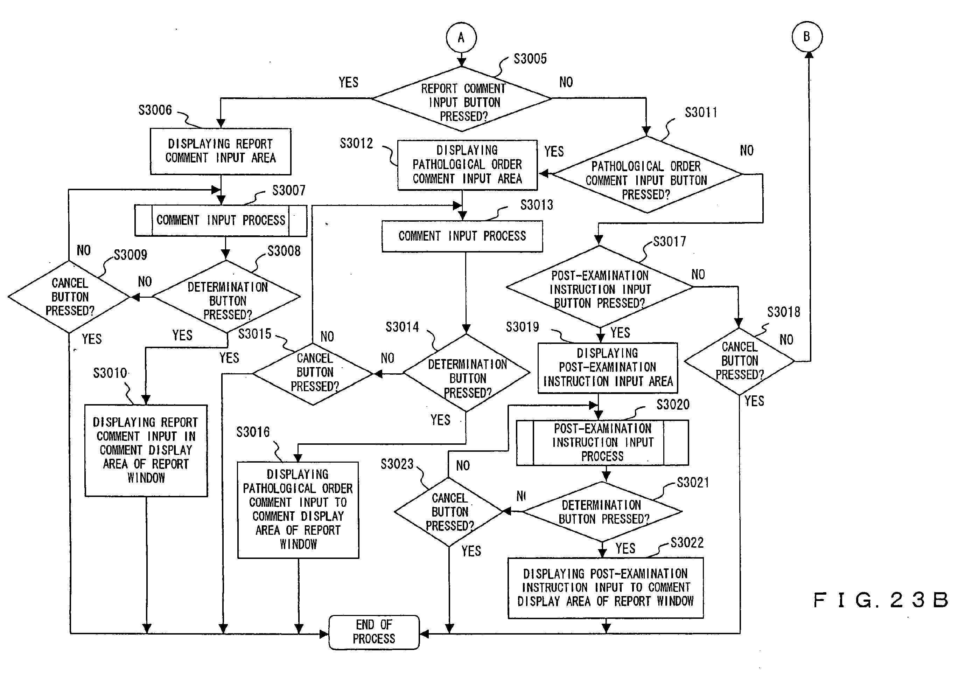

Described below are the processes shown in FIGS. 23A and 23B. FIGS. 23A and 23B show the flowcharts of the contents of the new comment input process in S3000 shown in FIG. 4A. In this process, comments generated as necessary depending on the entire examination result are newly input.

First, in S3001 shown in FIG. 23A, it is determined whether or not the new comment input button has been pressed. If the determination result is YES, control is passed to S3002. If the determination result is NO, the new comment input process is terminated as is, and control is returned to the process shown in FIG. 4A. The new comment input button is a character string "comment" displayed with an underline in the comment display area 65 (FIG. 5) of the report window screen.

In S3002, it is determined whether or not the pathological order flag described above in the processes in S1119 and S1120 (FIG. 12B) has been set ON. If the determination result is YES, control is passed to S3003. If the determination result is NO, control is passed to S3004.

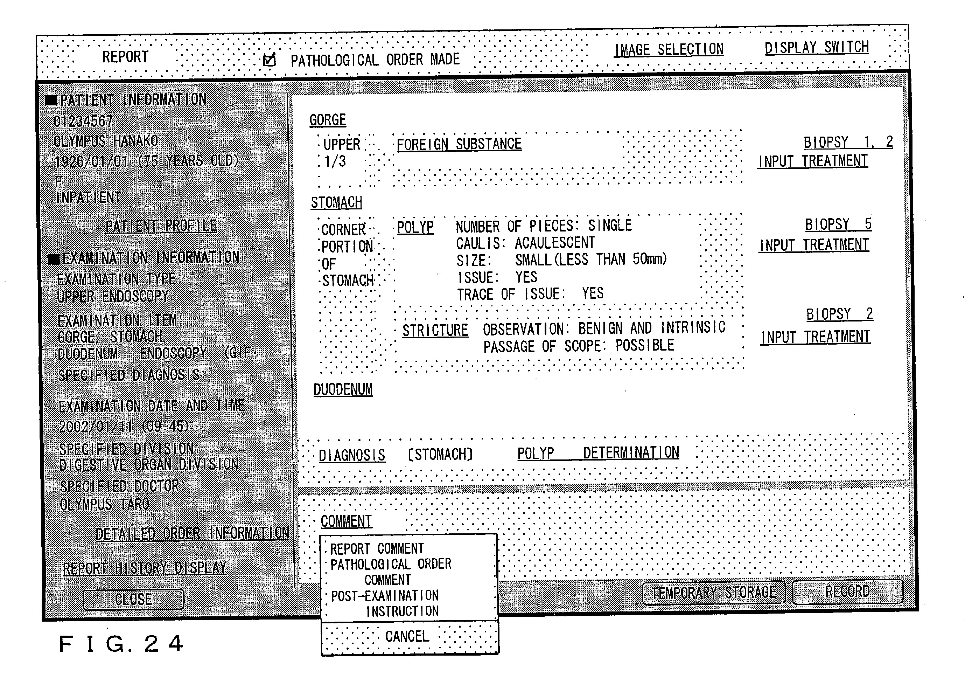

In S3003, the "report comment" input button for input of a comment relating to the diagnosis report, the "pathological order comment" input button for input of a comment added to a pathological order, the "post-examination instruction" input button for input of a comment as an instruction after an examination, and the "cancel" button for cancellation of the input of a comment are superposed on the report window screen displayed on the display unit 46 as a list of target items of comments. Then, control is passed to the processes in S3005 shown in FIG. 23B. FIG. 24 shows a display example.

In S3004, in the above-mentioned buttons, the "report comment" input button, the "post-examination instruction" input button, and the "cancel" button except the "pathological order comment" input button are superposed on the report window screen displayed on the display unit 46 as a list of target items of comments.

In S3005 shown in FIG. 23B, it is determined whether or not the "report comment" input button has been pressed in the list of the target items of comments. If the determination result is YES, control is passed to S3006. If the determination result is NO, control is passed to S3011.

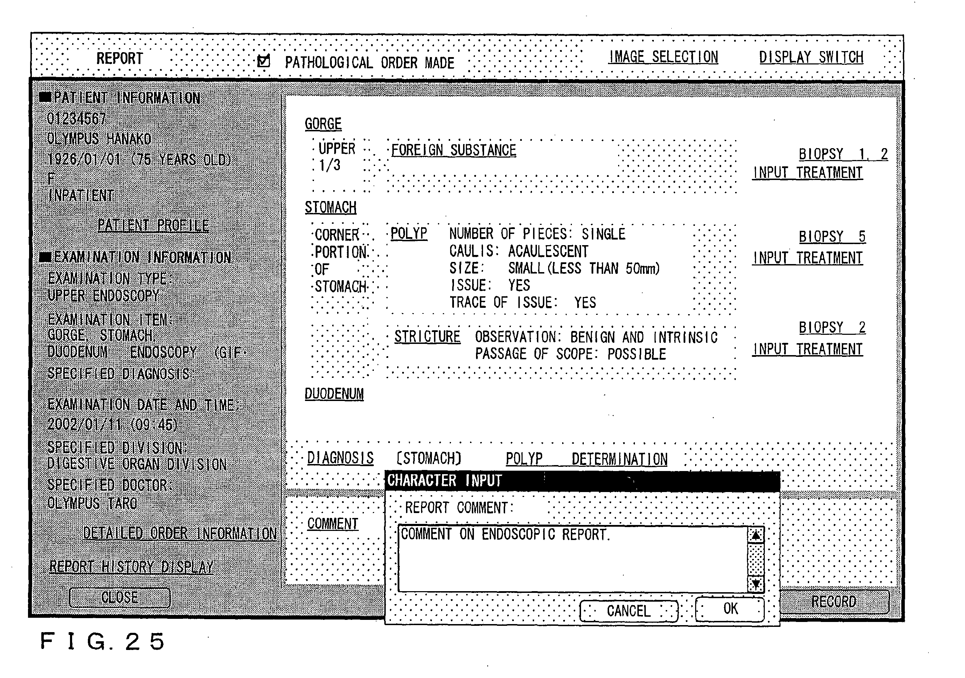

In S3006, the window screen having a report comment input area for input of a report comment is superposed on the report window screen. FIG. 25 shows a display example.

In S3007, a comment input process is performed. This process is performed by obtaining the contents of the operation of the keyboard, etc. of the input unit 45, and displaying the text corresponding to the contents of the operation, that is, the contents of the comment, in the report comment input area.

In S3008, it is determined whether or not the determination button (the "OK" button provided at the lower right portion of the window screen shown in FIG. 25) has been pressed. If the determination result is YES, control is passed to S3010. If the determination result is NO, control is passed to S3009.

In S3009, it is determined whether or not the cancel button (the "cancel" button at the lower right portion of the window screen shown in FIG. 25) has been pressed. If the determination result is YES, the new comment input process is terminated, and control is returned to the process in FIG. 4A. If the determination result is NO, control is passed to S3007, and the above-mentioned processes are repeated.

In S3010, the contents of the comment displayed in the report comment input area in the new comment input process in S3007 are displayed as a report comment in the comment display area 65 on the report window screen. Afterwards, the new comment input process is terminated, and control is returned to the process shown in FIG. 4A.

If the determination result is NO in S3005, it is determined in S3011 whether or not the "pathological order comment" input button in the list of the target items of comments has been pressed. If the determination result is YES, control is passed to S3012. If the determination result is NO, control is passed to S3017.

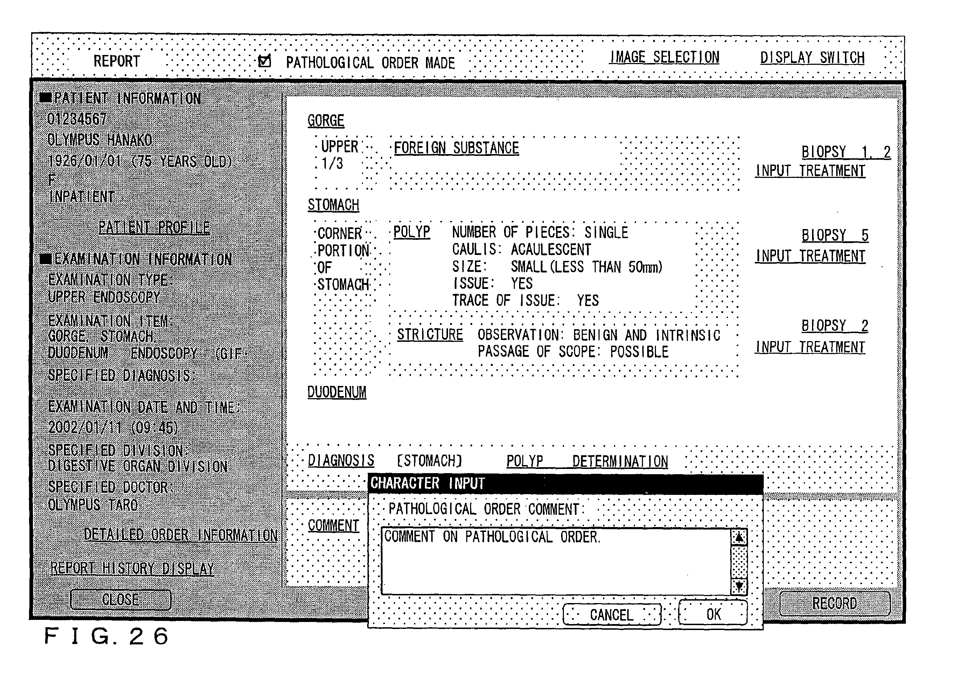

In S3012, the window screen having the pathological order comment input area for use in inputting a pathological order comment is superposed on the report window screen. FIG. 26 shows a display example.

In S3013, the comment input process similar to the above-mentioned process in S3007 is performed for the pathological order comment input area.

In S3014, it is determined whether or not the determination button (the "OK" button provided at the lower right of the window screen shown in FIG. 26) has been pressed. If the determination result is YES, control is passed to S3016. If the determination result is NO, control is passed to S3015.

In S3015, it is determined whether or not the cancel button (the cancel button to the left of the determination button at the lower right of the window screen shown in FIG. 26) has been pressed. If the determination result is YES, the new comment input process is terminated as is, and control is returned to the process shown in FIG. 4A. If the determination result is NO, control is returned to S3013, and the above-mentioned processes are repeated.

In S3016, the contents of the comment displayed in the pathological order comment input area in the comment input process in S3013 are displayed as a pathological order comment in the comment display area 65 of the report window screen. Then, the new comment input process is terminated, and control is passed to the process shown in FIG. 4A.

If the determination result is NO in S3011, it is determined in S3017 whether or not the "post-examination instruction" input button in the list of the target items of comments has been pressed. If the determination result is YES, control is passed to S3019. If the determination result is NO, control is passed to S3018.

In S3018, it is determined whether or not the cancel button (the "cancel" button in the bottom line of the list of the target items of comments shown in FIG. 24) has been pressed. If the determination result is YES, the new comment input process is terminated as is, and control is passed to the process shown in FIG. 4A. If the determination result is NO, control is returned to S3002 shown in FIG. 23A, and the above-mentioned processes are repeated.

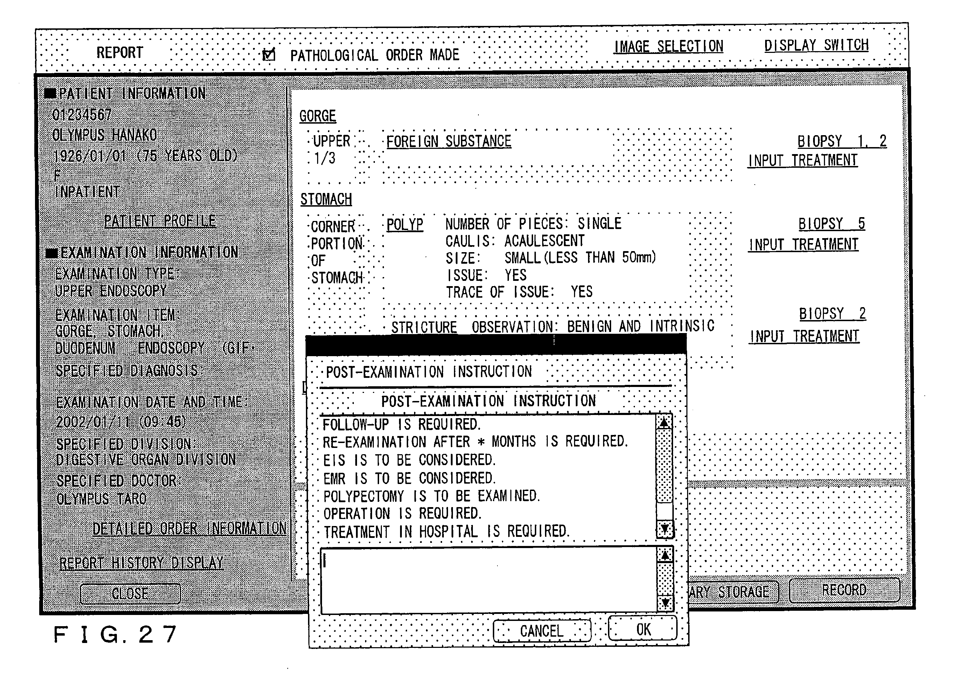

In S3019, the window screen having the post-examination instruction input area for use in inputting a post-examination instruction is superposed on the report window screen. FIG. 27 shows a display example. A list of fixed-form text of the post-examination instruction is displayed at the upper portion on the window screen shown in FIG. 27. The post-examination instruction input area is provided at the lower portion.

In S3020, the post-examination instruction input process is performed. In this process, the fixed-form text selected from the list shown at the upper portion of the window screen shown in FIG. 27 is displayed in the post-examination instruction input area, and the displayed fixed-form text is edited depending on the contents of the operation of the keyboard, etc. of the input unit 45, thereby generating text of the post-examination instruction. In this process, the laborious operation of generating text can be reduced.

In S3021, it is determined whether or not the determination button (the "OK" button provided at the lower right portion of the window screen shown in FIG. 27) has been pressed. If the determination result is YES, control is passed to S3022. If the determination result is NO, control is passed to S3023.

In S3022, the contents of the comment displayed in the post-examination instruction input area in the post-examination instruction input process in S3020 are displayed as a post-examination instruction in the comment display area 65 of the report window screen. Then, the new comment input process is terminated, and control is returned to the process shown in FIG. 4A.

In S3023, it is determined whether or not the cancel button (the "cancel" button to the left of the determination button at the lower right portion of the window screen shown in FIG. 27) has been pressed. If the determination result is YES, the new comment input process is terminated as is, and control is returned to the process shown in FIG. 4A. If the determination result is NO, control is returned to S3020, and the above-mentioned processes are repeated.

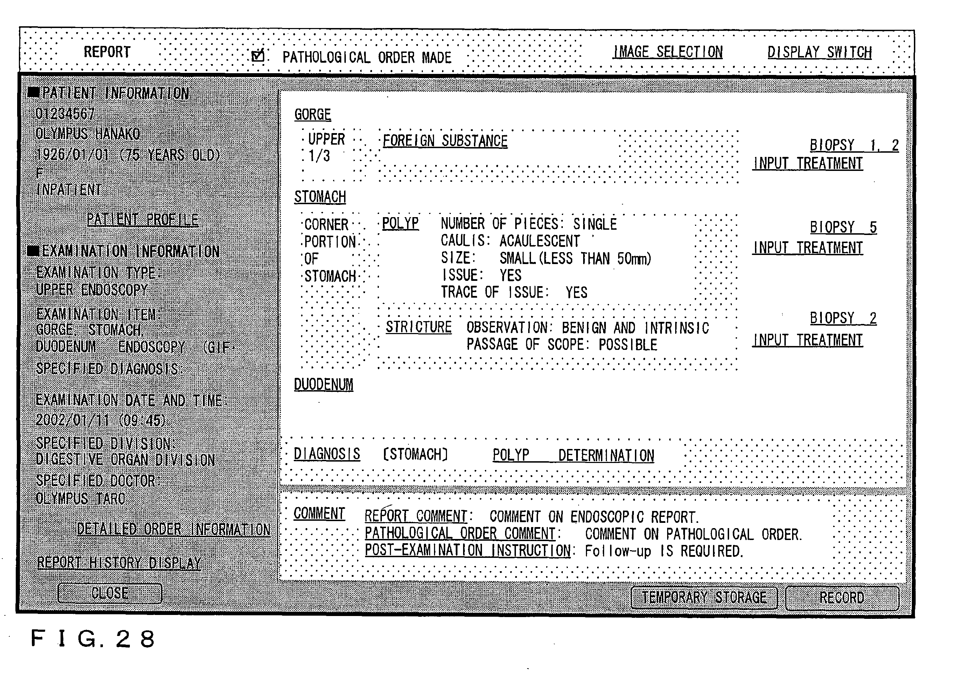

The above-mentioned processes are the new comment input process. FIG. 28 shows a display example of the report window screen after the process has been performed. A report comment, a pathological order comment, and a post-examination instruction are separately displayed in the comment display area 65 shown in FIG. 28.

The diagnosis report is completed in the above-mentioned processes. The processes described below are performed to edit and delete a generated diagnosis report.

In the explanation below, it is assumed that the new diagnosis input process is started with the report window screen on which data has been completely input to each of the observation/treatment display area 63, the diagnosis display area 64, and the comment display area 65 displayed on the display units 46 of the terminal devices 20-1, 20-2, and 20-3 as shown in FIG. 28.

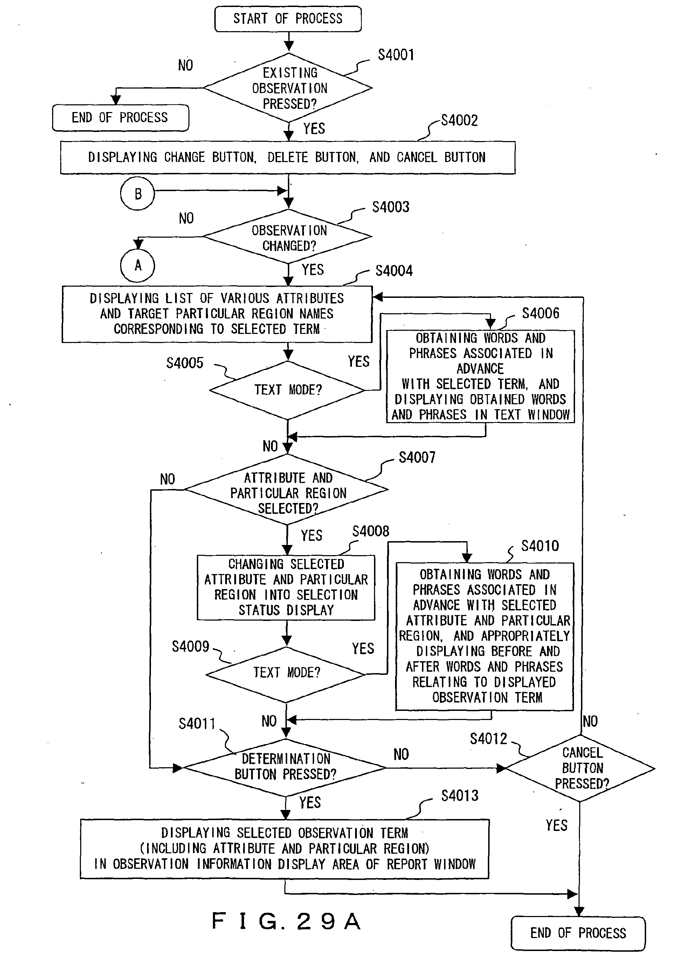

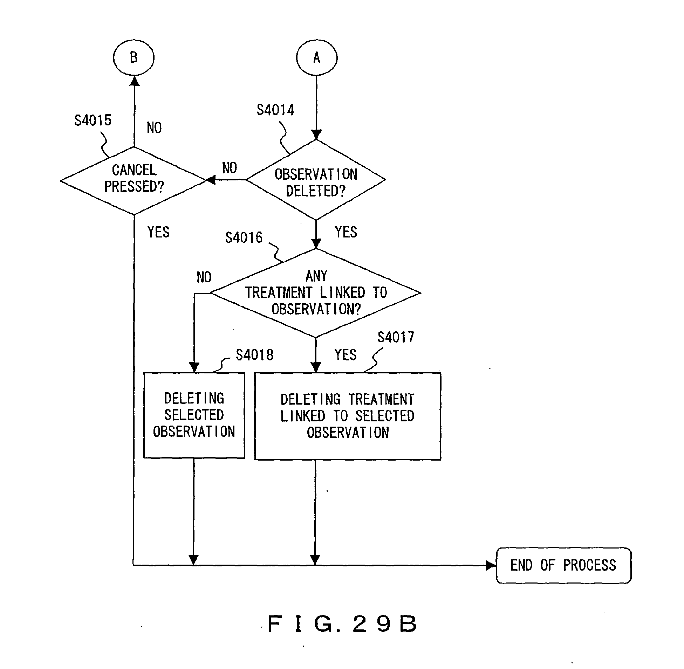

First described are the flowcharts shown in FIGS. 29A and 29B. FIGS. 29A and 29B are the flowcharts of the contents of the existing observation editing/deleting process in S4000 shown in FIG. 4A. The process is performed to edit and delete the contents of the observation of a diagnosis report and the corresponding treatment.

First, in S4001 shown in FIG. 29A, it is determined whether or not the character string forming the contents of the observation displayed in the observation/treatment display area 63 on the report window screen has been pressed. If the determination result is YES, control is passed to S4002. If the determination result is NO, the existing observation editing/deleting process is terminated as is, and control is returned to the process shown in FIG. 4A.

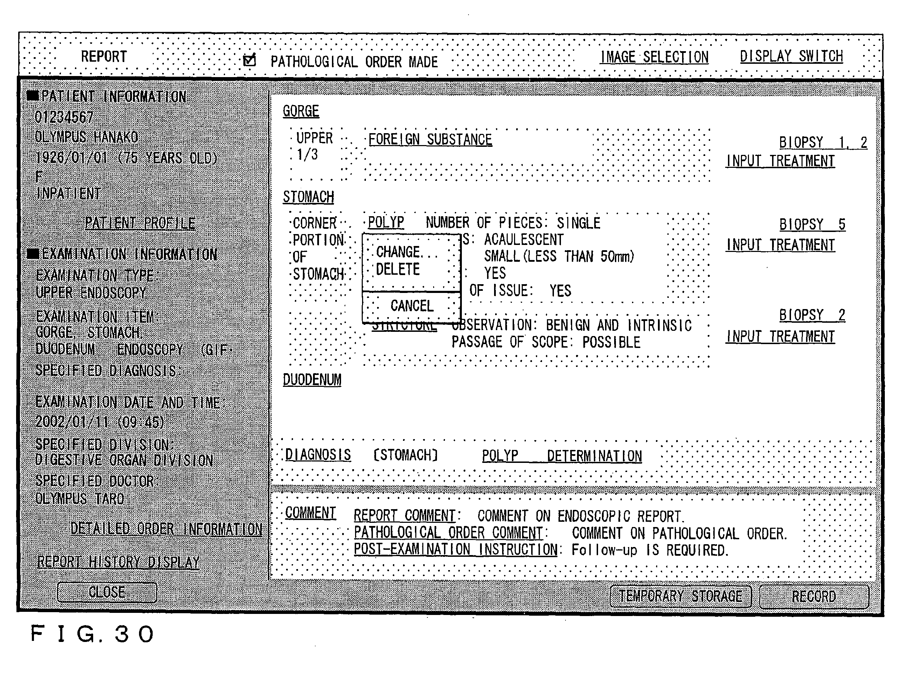

In S4002, a list of the edit instruction buttons formed by the "change" button, the "delete" button, and the "cancel" button is superposed on the report window screen displayed on the display unit 46 as buttons from which an instruction of the contents of the editing operation to be performed. FIG. 30 shows a display example. The list shown in FIG. 30 is displayed by detecting the press of the character string of the observation "polyp" about the region "corner portion of stomach" of the "stomach" displayed in the observation/treatment display area 63 on the report window screen shown in FIG. 28.

In S4003, it is determined whether or not the "change" button in the list of the edit instruction buttons has been pressed. If the determination result is YES, control is passed to S4004. If the determination result is NO, control is passed to S4014.

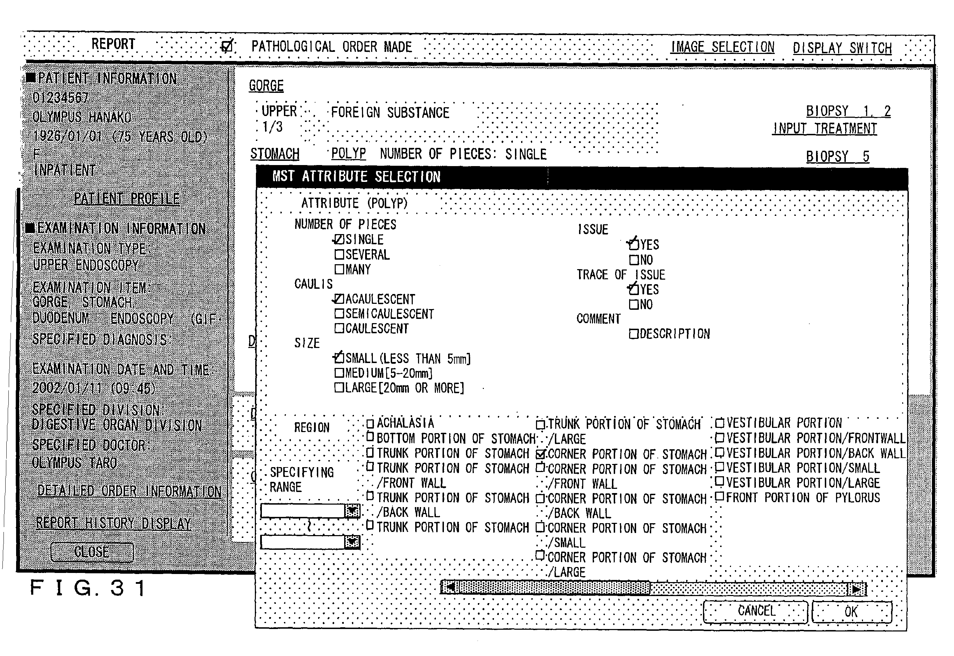

In S4004, a list of attributes and target particular regions corresponding to the selected observation term is superposed on the display unit 46. FIG. 31 shows a display example. The display example shown in FIG. 31 is an example of a list of attributes and target particular regions about the observation "polyp" about the "stomach", and the selection status about the attributes and target particular regions at the time is displayed by the check mark in the check box.

In S4005, it is determined whether or not the above-mentioned representation format mode is set as the "text mode". If the determination result is YES, the words and phrases associated in advance with the observation term selected from the list are obtained, and the obtained words and phrases (portion of text formed by a term or a combination of its derivative, a preposition, an auxiliary word, etc.) are displayed in the text window in S4006.

In S4007, it is determined whether or not an attribute and a target particular region have been selected from the list shown in FIG. 31. If the determination result is YES, control is passed to S4005. If the determination result is NO, control is passed to S4011.

In S4008, the display of the attributes and target particular regions selected in the preceding step of the process is changed into the selection status display. In the example shown in FIG. 31, a check mark is given to the check box corresponding to the display of the selected attribute and target particular region.

In S4009, it is determined whether or not the representation format mode described later is set as the text mode. If the determination result is YES, the words and phrases associated in advance with the attribute and the target particular region selected from the list are obtained, and the obtained words and phrases are appropriately arranged before and after the words and phrases associated with the observation term and displayed in the text window, thereby generating text.

In S4011, it is determined whether or not the determination button (the "OK" button provided at the lower right portion of the list of various attributes and target particular regions shown in FIG. 31) has been pressed. If the determination result is YES, control is passed to S4013. If the determination result is NO, control is passed to S4012.

In S4012, it is determined whether or not the cancel button (the "cancel" button to the left of the determination button at the lower right portion of the screen shown in FIG. 31) has been pressed. If the determination result is YES, the existing observation editing/deleting process is terminated, and control is returned to the process shown in FIG. 4A. If the determination result is NO, control is returned to S4004, and the above-mentioned processes are repeated.

In S4013, the observation terms, the attributes, and the target particular regions selected in the preceding processes are displayed as the contents of the observation in the observation/treatment display area 63 on the report window screen.

If the determination result is NO in S4003, it is determined in S4014 whether or not the "delete" button in the list of the above-mentioned edit instruction buttons has been pressed. If the determination result is YES, control is passed to S4016. If the determination result is NO, control is passed to S4015.

In S4015, it is determined whether or not the "cancel" button in the list of the above-mentioned edit instruction buttons has been pressed. If the determination result is YES, the existing observation editing/deleting process is terminated, and control is returned to the process shown in FIG. 4A. If the determination result is NO, control is returned to S4003 shown in FIG. 29A, and the above-mentioned processes are repeated.

In S4016, it is determined whether or not there is the information about the treatment associated (linked) with the selected observation term. If the determination result is YES, both the contents of the observation relating to the selected observation term and the treatment linked to the observation are deleted in S4017. If the determination result is NO, the contents of the observation relating to the selected observation term are deleted in S4018. Then, the existing observation editing/deleting process is terminated, and control is returned to FIG. 4A.

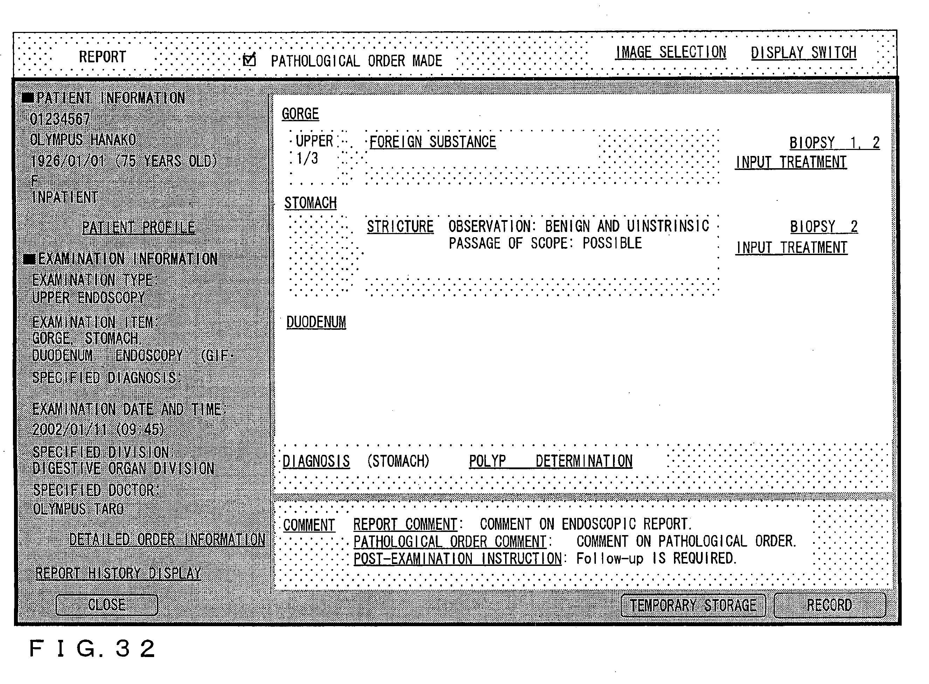

FIG. 32 shows an example of a report window screen in which a part of the observation has been deleted in the process in S4017 or S4018. As clearly indicated by the comparison with the data shown in FIG. 28, the contents of the observation relating to the observation "polyp" about the examination item "stomach", and the corresponding treatment "biopsy 5" have been deleted.

The above-mentioned process is the existing observation editing/deleting process.

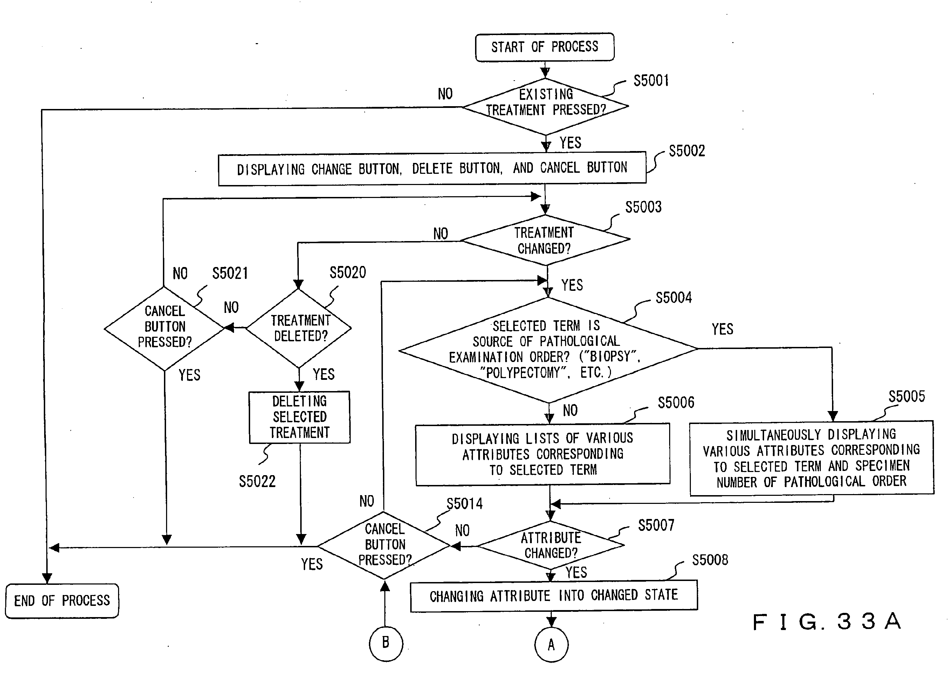

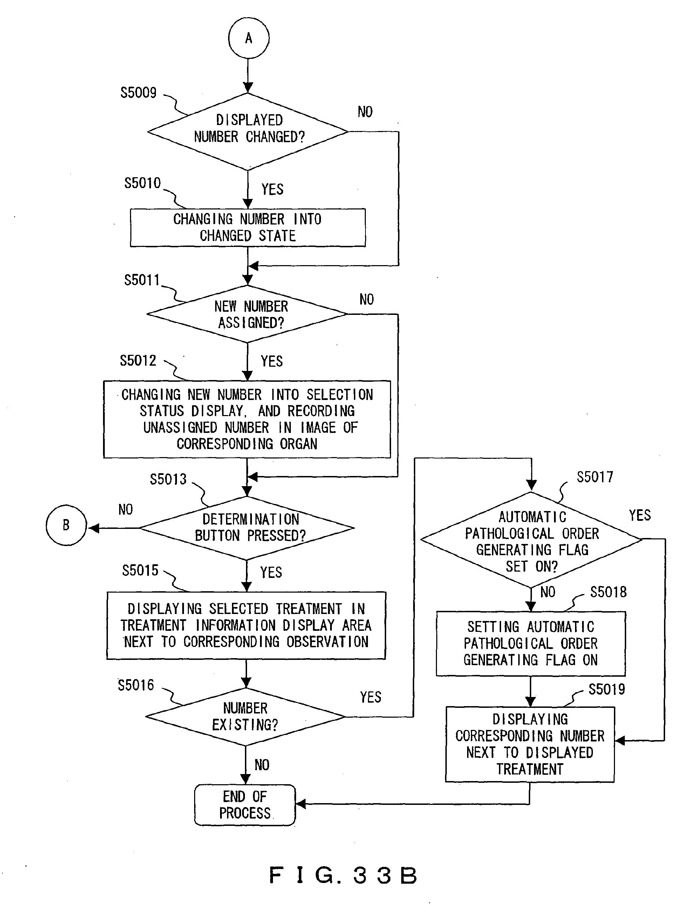

The processes shown in FIGS. 33A and 33B are described below. These figures are the flowcharts of the existing treatment editing/deleting process performed in S5000 shown in FIG. 4A. The process is performed to edit and delete the treatment linked to the observation without changing the contents of the observation in a diagnosis report.

First, in S5001, it is determined whether or not the character string indicating the contents of the treatment displayed in the observation/treatment display area 63 of the report window screen has been pressed. If the determination result is YES, control is passed to S5002. If the determination result is NO, the existing treatment editing/deleting process is terminated as is, and control is returned to the process shown in FIG. 4A.

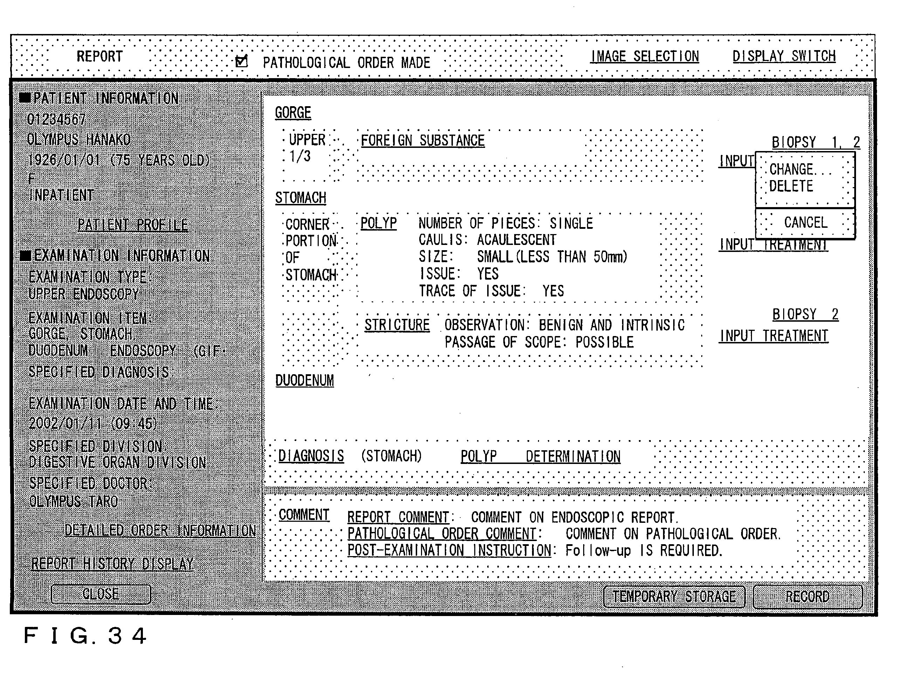

In S5002, a list of the edit instruction buttons formed by the "change" button, the "delete" button, and the "cancel" button are superposed as the buttons for selecting the instruction of the contents of the editing operation to be performed on the report window screen displayed on the display unit 46. FIG. 34 shows a display example. The display of the list shown in FIG. 34 is displayed by pressing the "biopsy 1, 2" displayed in the observation/treatment display area 63 on the report window screen shown in FIG. 28 and linked to the observation "foreign substance" about the "gorge".

In S5003, it is determined whether or not the "change" button in the list of the edit instruction buttons has been pressed. If the determination result is YES, control is passed to S5004. If the determination result is NO, control is passed to S5020.

In S5004, it is determined whether or not a predetermined treatment term has been selected in advance as a source of the above-mentioned pathological examination order. If the determination result is YES, control is passed to S5005. If the determination result is NO, control is passed to S5006.

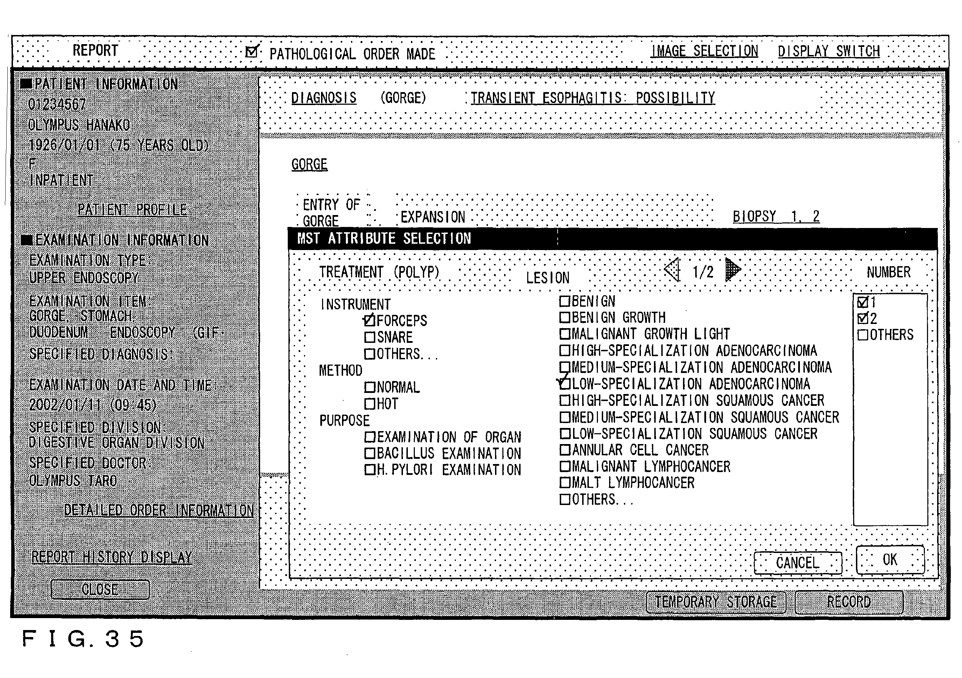

In S5005, the window screen having a list of various attributes corresponding to the treatment terms selected from the list and an input column of an above-mentioned specimen number is superposed on the display unit 46. FIG. 35 shows a display example. The display example shown in FIG. 35 shows an example of a window screen having a list of attributes about the "biopsy" selected from the list of treatment terms about the "gorge" and an input column of a specimen number. In this display example, the selection status about the attribute and the specimen number at the time point is indicated by the check mark given to the check box.

In S5006, a list of various attributes corresponding to the treatment term selected from the list is superposed on the display unit 46.

In S5007, it is determined whether or not the attribute has been selected from the list displayed in the process in S5005 or S5006. If the determination result is YES, control is passed to S5008. If the determination result is NO, control is passed to S5014.

In S5008, the display of the attribute selected in the process in the preceding step is changed into the selection status display. In the example shown in FIG. 35, a check mark is given to the check box corresponding to the display of the selected attribute.

In FIG. 33B, in S5009, it is determined whether or not a number displayed in the input column of the specimen number of the window screen displayed on the display unit 46 has been selected. If the determination result is YES, the display is changed into a display indicating the selected number in S5010.

In S5011, it is determined whether or not a new number has been selected. If the determination result is YES, the display of the number is changed into the display indicating that the numeric display is a specimen number in S5012. If there is image data of the organ from which the specimen has been obtained, the specimen number is displayed in the position of the specimen in the image of the organ.

In S5013, it is determined whether or not the determination button (the "OK" button at the lower right portion on the screen shown in FIG. 35) has been pressed. If the determination result is YES, control is passed to S5015. If the determination result is NO, control is passed to S5014 shown in FIG. 33A.

Back in FIG. 33A, in S5014, it is determined whether or not the cancel button (the "cancel" button shown at the lower right portion of the screen shown in FIG. 35) has been pressed. If the determination result is YES, the existing treatment editing/deleting process is terminated, and control is returned to the process in FIG. 4A. If the determination result is NO, control is returned to S5004, and the above-mentioned processes are repeated.

Back in S5015 shown in FIG. 33B, the selected treatment term and the attribute in the preceding processes are displayed in the position in which the contents of the treatment has been pressed in the display area of the treatment information in the observation/treatment display area 63 of the report window screen.

In S5016, it is determined whether or not a specimen number has been selected. If the determination result is YES, control is passed to S5017. If the determination result is NO, the existing treatment editing/deleting process is terminated, and control is returned to the process shown in FIG. 4A.

In S5017, it is determined whether or not the above-mentioned automatic pathological order generating flag has been set ON. If the determination result is NO, the automatic pathological order generating flag is set ON in S5018.

In S5019, the selected specimen number is displayed in the position to the right of the word and phrase relating to the treatment and displayed in the observation/treatment display area 63 of the report window screen in the above-mentioned process in S5015. Then, the existing treatment editing/deleting process is terminated, and control is returned to the process shown in FIG. 4A.

If the determination result is NO in S5003 shown in FIG. 33A, it is determined in S5020 whether or not the "delete" button in the list of the above-mentioned edit instruction buttons has been pressed. If the determination result is YES, control is passed to S5022. If the determination result is NO, control is passed to S5021.

In S5021, it is determined whether or not the "cancel" button in the list of the above-mentioned edit instruction buttons has been pressed. If the determination result is YES, the existing treatment editing/deleting process is terminated, and control is returned to the process shown in FIG. 4A. If the determination result is NO, control is returned to S5003, and the above-mentioned processes are repeated.

In S5022, the contents of the treatment relating to the selected treatment term are deleted. Then, the existing treatment editing/deleting process is terminated, and control is returned to the process shown in FIG. 4A.

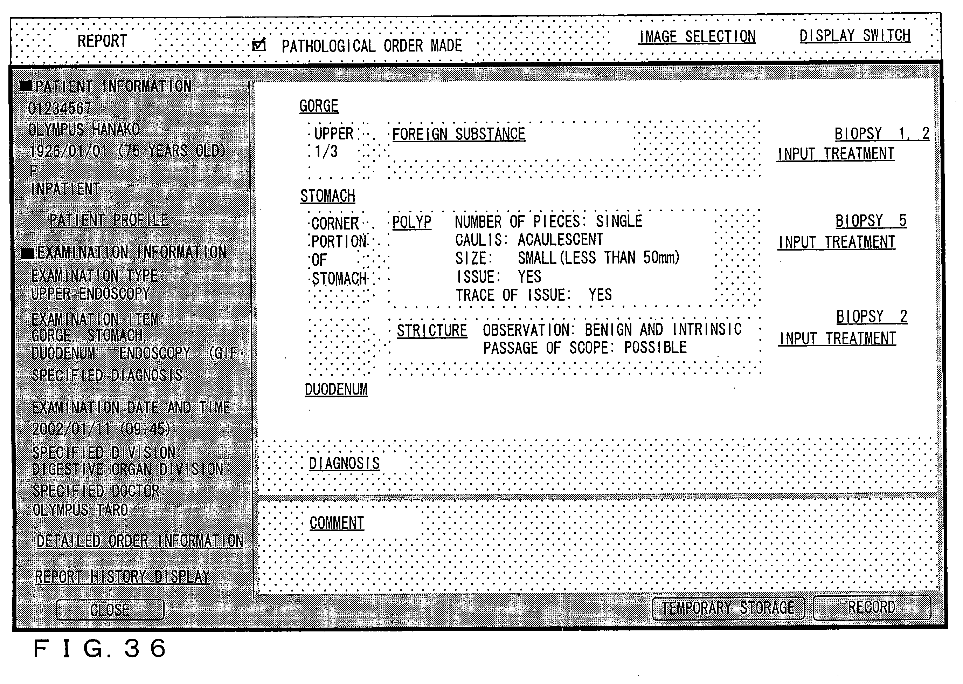

FIG. 36 shows an display of the report window screen after a part of the treatment has been deleted in the process in S5022. As clearly indicated by comparing the screen sown in FIG. 36 with the screen shown in FIG. 28, the "biopsy 1, 2" corresponding to the observation "foreign substance" about the examination item "gorge" has been deleted.

The above-mentioned processes are the existing treatment editing/deleting process.

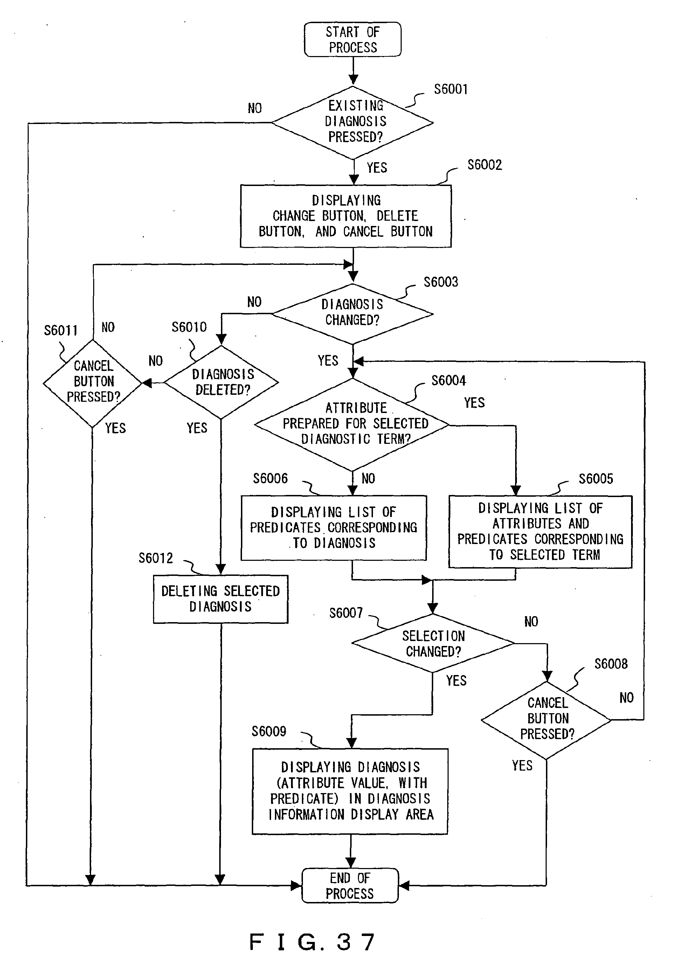

Described below is the flowchart shown in FIG. 37. FIG. 37 is a flowchart of the existing treatment editing/deleting process performed in S6000 shown in FIG. 4A. In this process, the contents of the diagnosis in the diagnosis report are edited and deleted.

First, in S6001, it is determined whether or not the character string forming the contents of the diagnosis and displayed in the diagnosis display area 64 on the report window screen has been pressed. If the determination result is YES, control is passed to S6002. If the determination result is NO, the existing diagnosis editing/deleting process is terminated as is, and control is returned to the process shown in FIG. 4A.

In S6002, a list of the edit instruction buttons formed by the "change" button, the "delete" button, and the "cancel" button as shown in FIGS. 30 and 34 is superposed on the report window screen displayed on the display unit 46.

In S6003, it is determined whether or not the "change" button in the list of the edit instruction buttons has been pressed. If the determination result is YES, control is passed to S6004. If the determination result is NO, control is passed to S6010.

In S6004, it is determined whether or not a list of various attributes corresponding to the pressed diagnostic term has been prepared. If the determination result is YES, control is passed to S6005. If the determination result is NO, control is passed to S6006.

In S6005, the attributes corresponding to the pressed diagnostic term and a list of the predicates corresponding to the diagnostic term are superposed on the display unit 46. Then, control is passed to S6007.

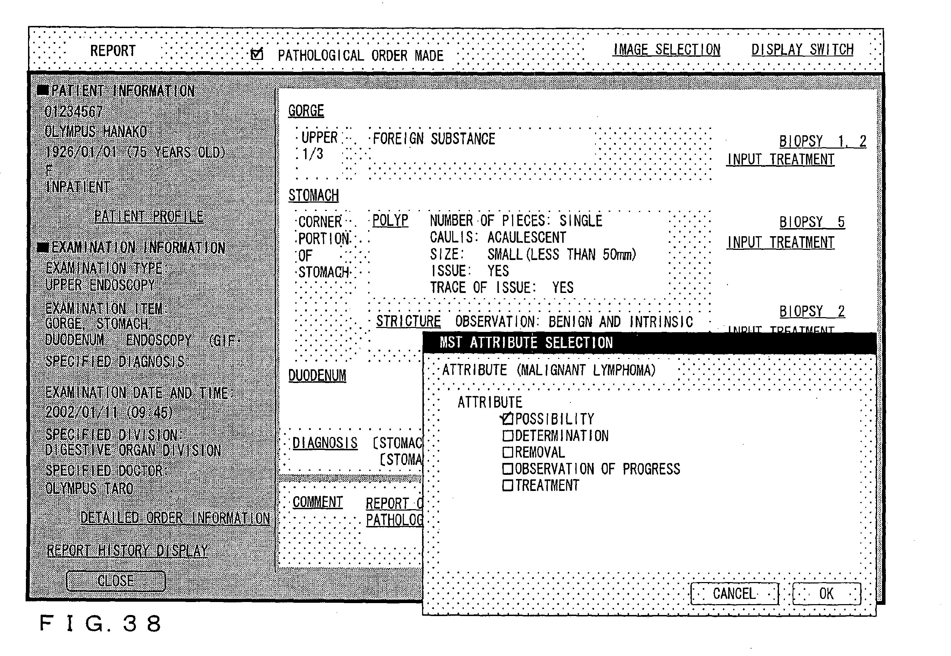

In S6006, a list of the predicates corresponding to the pressed diagnostic term is superposed on the display unit 46. FIG. 38 shows a display example. The display example shown in FIG. 38 shows a list of the predicates about the diagnostic term "malignant lymphoma" about the "stomach", and the selection status of the predicate at the time is indicated by the check mark given to the check box. In the display example shown in FIG. 38, the character string "attribute" is displayed, but the list shown in FIG. 38 is processed as predicates in the system.

In S6007, it is determined whether or not selected contents have been changed in a list of attributes or a list of predicates displayed on the display unit 46. If the determination result is YES, control is passed to S6008. If the determination result is NO, control is passed to S6009.

In S6008, it is determined whether or not the cancel button (the "cancel" button displayed on the window screen of the attribute list or the predicate list) has been pressed. If the determination result is YES, the existing diagnosis editing/deleting process is terminated, and control is returned to the process in FIG. 4A. If the determination result is NO, control is returned to S6004, and the above-mentioned processes are repeated.

In S6009, the selected diagnostic term, attribute, and predicate are associated with a title of an examination item, and displayed in the diagnosis display area 64 of the report window screen. Then, the existing diagnosis editing/deleting process is terminated, and control is returned to the process in FIG. 4A.

If the determination result in S6003 is NO, it is determined in S6010 whether or not the "delete" button in the list of the above-mentioned edit instruction buttons has been pressed. If the determination result is YES, control is passed to S6012. If the determination result is NO, control is passed to S6011.

In S6011, it is determined whether or not the "cancel" button in the list of the above-mentioned "report comment" input buttons has been pressed. If the determination result is YES, the existing diagnosis editing/deleting process is terminated, and control is returned to the process shown in FIG. 4A. If the determination result is NO, control is returned to S6003, and the above-mentioned processes are repeated.

In S6012, the contents of the treatment relating to the selected diagnostic term are deleted. Then, the existing diagnosis editing/deleting process is terminated, and control is returned to the process shown in FIG. 4A.

The above-mentioned processes are the existing diagnosis editing/deleting process.

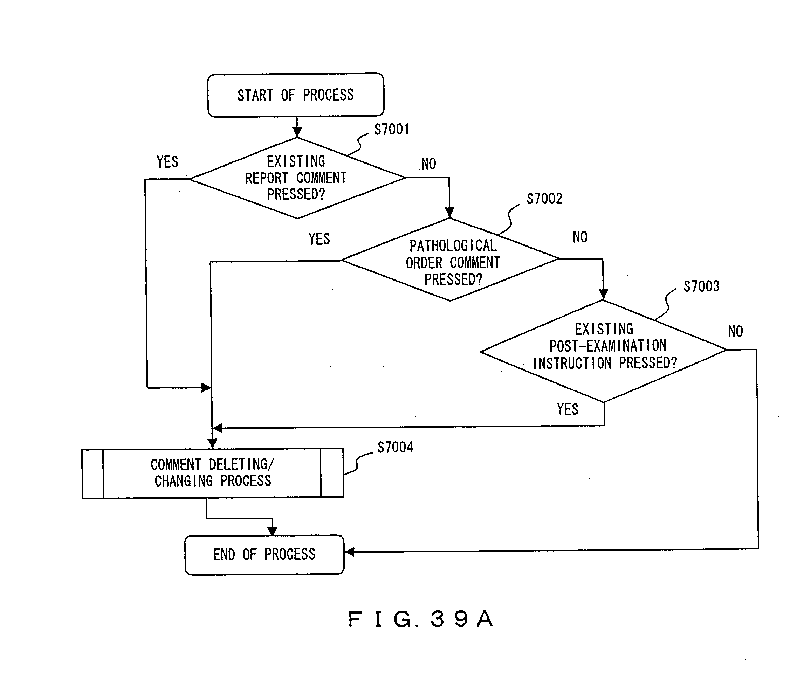

Described below is the flowchart shown in FIG. 39A. FIG. 39A is a flowchart of the contents of the existing comment editing/deleting process performed in S7000 shown in FIG. 4A. The process is performed to edit and delete the contents of the comment in a diagnosis report.

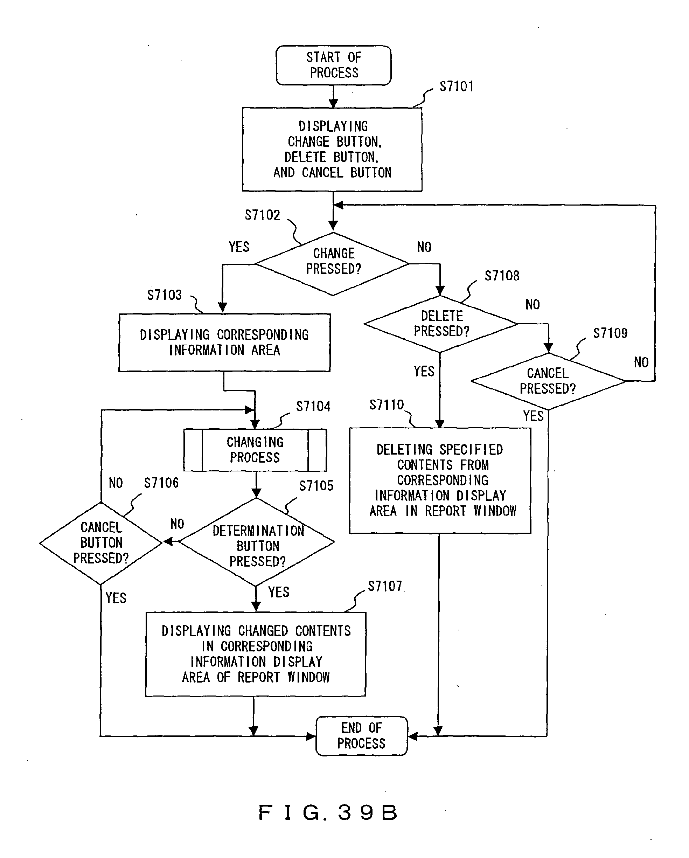

First, in S7001, it is determined whether or not a character string relating to the comment report displayed in the comment display area 65 on the report window screen has been pressed. In S7002, it is determined whether or not a character string relating to the pathological order comment has been pressed. In S7003, it is determined whether or not a character string relating to the post-examination instruction has been pressed. If any of the determination results in S7001, S7002, and S7003 is YES, the comment deleting/changing process displayed in the flowchart shown in FIG. 39B is performed in S7004.

After performing the process in S7004, or if the determination results are NO in all processes in S7001, S7002, and S7003, the existing comment editing/deleting process is terminated, and control is returned to the process shown in FIG. 4A.

Described below is the comment deleting/changing process shown in FIG. 39B.

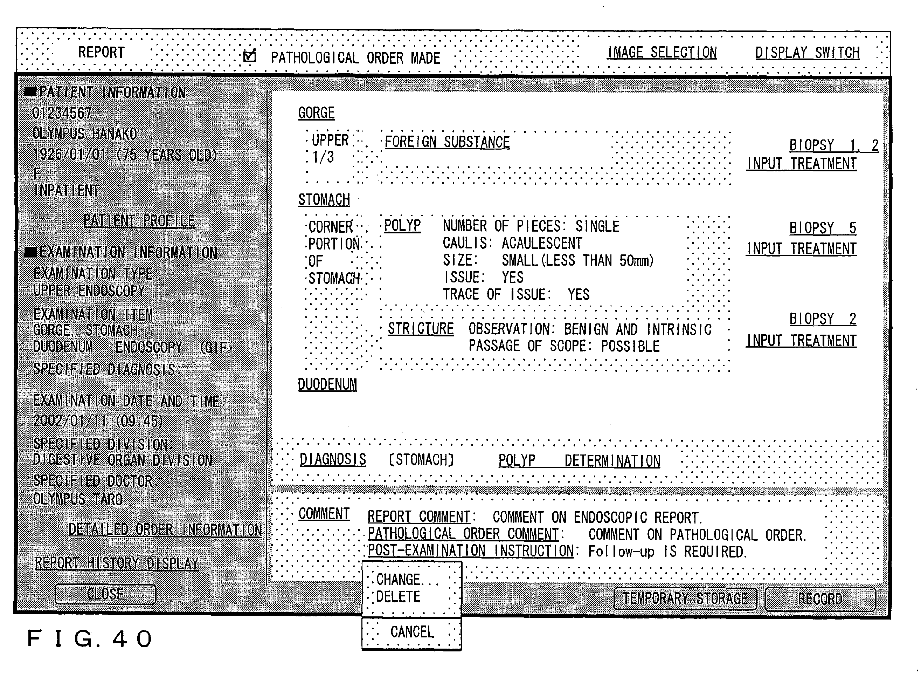

First, in S7101, a list of the edit instruction buttons formed by the "change" button, the "delete" button, and the "cancel" button is superposed on the report window screen displayed on the display unit 46. FIG. 40 shows a display example. The display of a list shown in FIG. 40 is made by pressing the character string "post-examination instruction" displayed in the comment display area 65 on the report window screen shown in FIG. 28.

In S7102, it is determined whether or not the "change" button in the list of the edit instruction buttons has been pressed. If the determination result is YES, control is passed to S7103. If the determination result is NO, control is passed to S7108.

In S7103, the window screen having a comment input area in which data can be input for the item to be commented on and pressed in the process in S7001, S7002, or S7003 shown in FIG. 39A is superposed.

In S7104, the changing process is performed. In this process, the contents of the operation relating to the keyboard, etc. of the input unit 45 are obtained, the contents of the comment displayed in the comment input area are changed based on the contents of the operation, and the result is displayed in the comment input area.

In S7105, it is determined whether or not the determination button (the "OK" button provided on the window screen having the comment input area) has been pressed. If the determination result is YES, control is passed to S7107. If the determination result is NO, control is passed to S7106.

In S7106, it is determined whether or not the cancel button (the "cancel" button provided on the window screen having the comment input area) has been pressed. If the determination result is YES, the comment deleting/changing process is terminated as is, and control is returned to the process shown in FIG. 39A. If the determination result is NO, control is returned to S7104, and the above-mentioned processes are repeated.

In S7107, the contents of the comment displayed in the comment input area in the comment changing process in S7104 are displayed in the comment display area 65 on the report window screen as a comment on the target item pressed in any of the processes in S7001, S7002, and S7003 shown in FIG. 39A. Then, the comment deleting/changing process is terminated, and control is returned to the process in FIG. 39A.

If the determination result in S7102 is NO, it is determined in S7108 whether or not the "delete" button in the list of the above-mentioned edit instruction buttons has been pressed. If the determination result is YES, control is passed to S7110. If the determination result is NO, control is passed to S7109.

In S7109, it is determined whether or not the "cancel" button in the list of the above-mentioned edit instruction buttons has been pressed. If the determination result is YES, the comment deleting/changing process is terminated, and control is returned to the process in FIG. 39A. If the determination result is NO, control is returned to the process in S7102, and the above-mentioned processes are repeated.

In S7110, the display of the comment on the target item pressed in any of the processes in S7001, S7002, and S7003 is deleted from the comment display area 65 on the report window screen. Then, the comment deleting/changing process is terminated, and control is returned to the process shown in FIG. 39A.

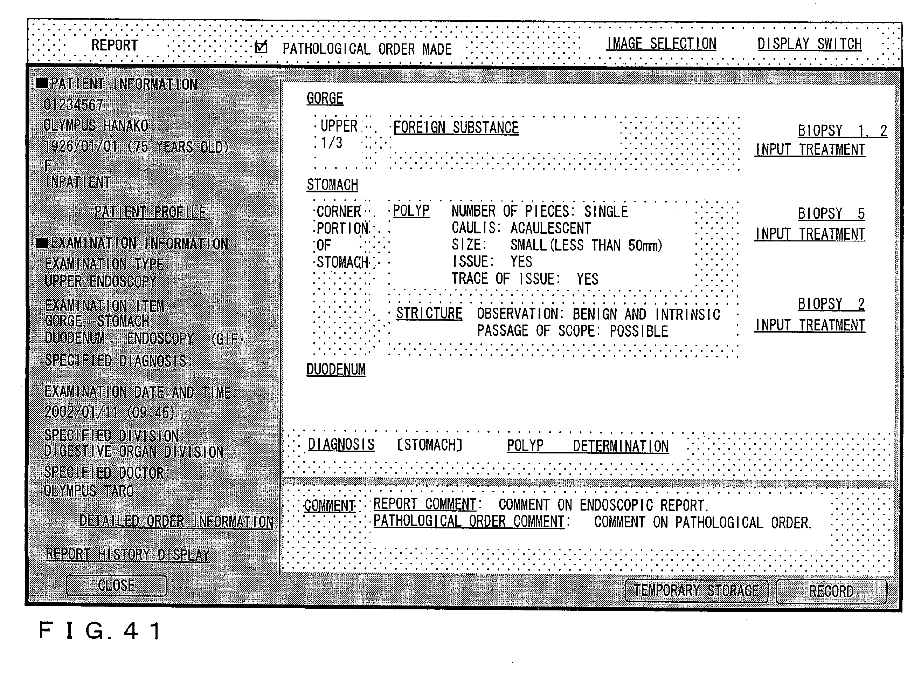

FIG. 41 shows an example of the report window screen after a part of the comment has been deleted in the process in S7110. As clearly indicated by comparing the screen shown in FIG. 41 with the screen shown in FIG. 28, the comment on the "post-examination instruction" has been deleted.

The above-mentioned processes are the existing comment editing/deleting process.

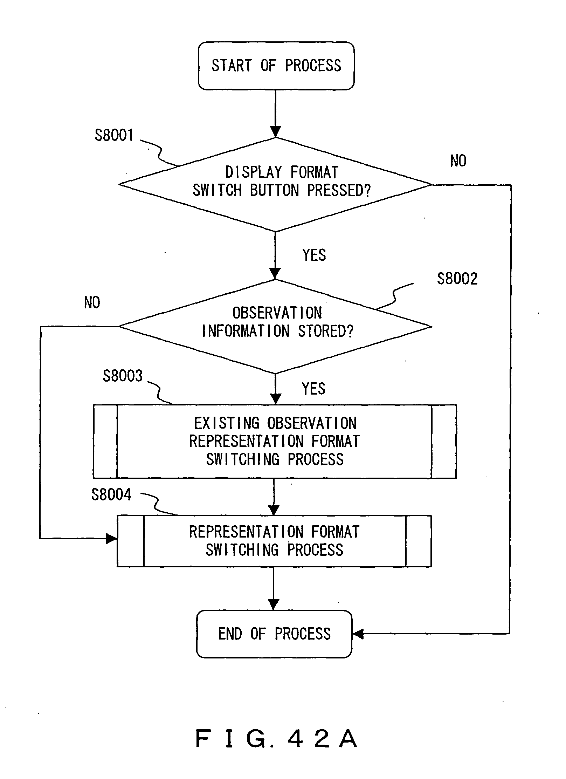

Described below is the flowchart shown in FIG. 42A. FIG. 42A is a flowchart of the observation representation format switching process performed in S8000 shown in FIG. 4A. In this process, the representation format mode for setting the representation format of the contents of the observation in a diagnosis report to be generated can be switched between the itemization mode in which observation terms and words and phrases relating to the observation terms are listed, and the above-mentioned text mode in which the observation terms and the words and phrases relating to the observation terms are displayed by text.

In FIG. 42A, in S8001, it is determined whether or not a display format switch button has been pressed. If the determination result is YES, control is passed to S8002. If the determination result is NO, the observation representation format switching process is terminated as is, and control is returned to the process shown in FIG. 4A. The display format switch button refers to an underlined character string "display switch" at the upper right corner of the report window screen shown in FIG. 5.

In S8002, it is determined whether or not observation information has been stored, that is, whether or not the contents of any observation has been input. If the determination result is YES, control is passed to S8003. If the determination result is NO, control is passed to S8004.

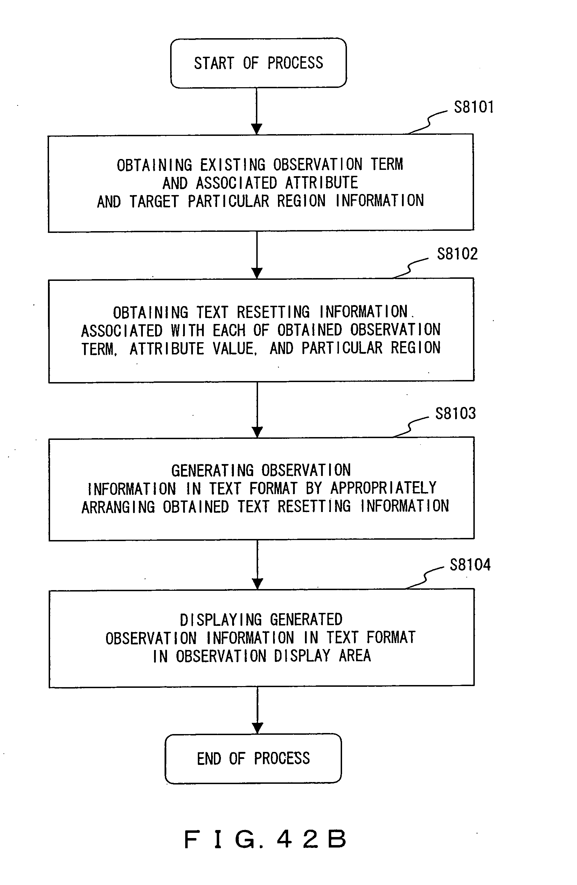

In S8003, the existing observation representation format switching process shown in the flowchart in FIG. 42B is performed.

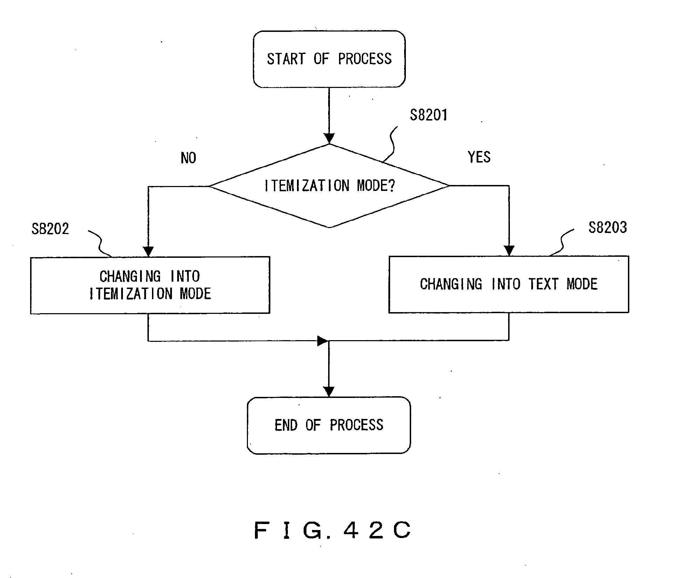

In S8004, the representation format switching process shown in the flowchart shown in FIG. 42C is performed. Then, the observation representation format switching process is terminated, and control is returned to the process shown in FIG. 4A.

Described below is the existing observation representation format switching process shown in FIG. 42B.

In S8101, the information about the observation term and the attribute and target particular region associated with the observation term is obtained from the contents of the observation detected in the process in S8002 shown in FIG. 42A.

In S8102, the text resetting information (about the above-mentioned words and phrases) associated in advance with each of the obtained observation terms, attributes, and target particular regions is obtained. In the subsequent S8103, the observation terms, attribute, target particular regions, and the text resetting information are appropriately arranged to generate text representing the contents of the observation. The processes in S8102 and S8103 are similar to the processes in S1008 and S1013 in the above-mentioned new observation input process (FIGS. 6A and 6B).

In S8104, the contents of the observation by the generated text are displayed in the observation/treatment display area 63 of the report window screen. Then, the existing observation representation format switching process is terminated, and control is returned to the process shown in FIG. 42A.

Described below is the representation format switching process shown in FIG. 42C.

First, in S8201, it is determined whether or not the current setting of the above-mentioned representation format mode is the itemization mode. If the determination result is YES, the setting of the representation format mode is changed into the text mode in S8202. On the other hand, if the determination result is NO, the setting of the representation format mode is changed into the itemization mode in S8203. After completing the process in S8202 or S8203, the representation format switching process is terminated, and control is returned to the process shown in FIG. 42A.

By performing each of the above-mentioned processes in the system shown in FIG. 2A, a diagnosis report can be generated, edited, and recorded in this system.

The present invention can be embodied by generating a program to be executed by a computer having a standard configuration and allowing the computer to perform each of the above-mentioned processes. The above-mentioned program can be stored in a computer-readable recording medium and can be read by the computer and executed by the CPU, thereby realizing the present invention.

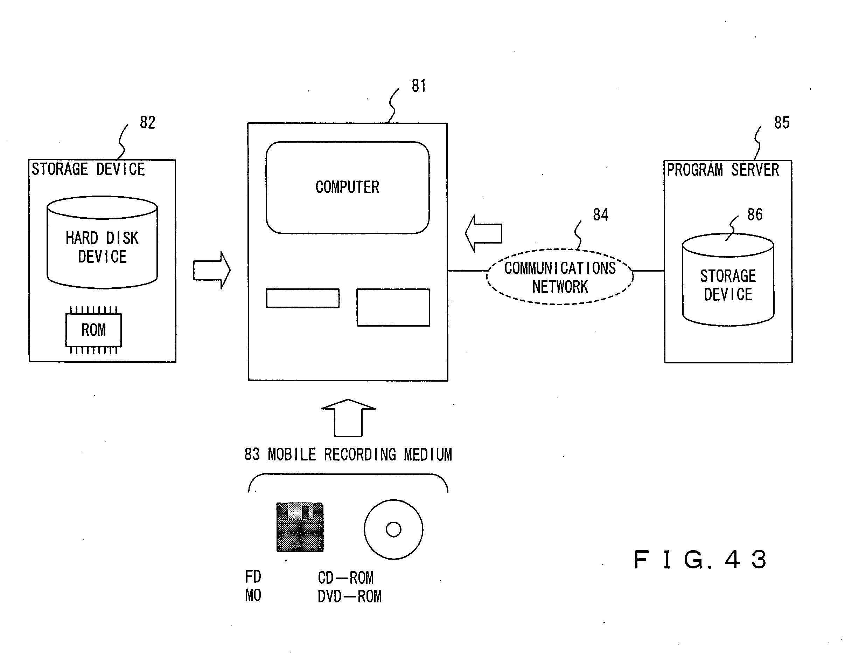

FIG. 43 shows an example of a computer-readable recording medium storing a control program. The recording medium can be, for example, a storage device 82 such as ROM, a hard disk device, etc. provided inside or outside a computer 81, and a mobile recording medium 83 such as an FD (flexible disk), an MO (magneto-optical disk), CD-ROM, DVD-ROM, etc.

A recording medium can be a storage device 86 provided inside or outside a program server 85 connected to the computer 81 through a communications network 84. In this case, a transmission signal obtained by modulating a carrier wave by a control program recorded in the storage device 86 of the program server 85 can be transmitted, and the computer 81 demodulates the control program from the transmission signal received through the communications network 84 to allow the CPU to execute the program.

Furthermore, the present invention can be realized as various improved and amended embodiments without limiting the applications to the above-mentioned embodiments.

For example, on the various screens according to the above-mentioned embodiment, the character string which can be clicked with the mouse is underlined. This can be replaced with a character string in a different color from normal character strings, or the character string can be displayed with an icon.

As described above in detail, according to the present invention, the observation on an examination result can be displayed on a display device, a request to input the treatment information about the treatment based on the observation is issued, the treatment information is input in response to the request, the observation information about the observation and the corresponding treatment information are associated and stored, thereby generating a diagnosis report.

Thus, the interference with the normal flow of thought by the investigation of the treatment after the observation can be eliminated. Therefore, an uncomfortable feeling of a report maker in hand-writing a diagnosis report can be avoided.

## Claims

1. A system for use in generating a diagnosis report, comprising:
an observation display unit (1) displaying observation on an examination result;
a treatment information input request notification unit (2) transmitting a notification of a request to input treatment information about the treatment in response to the observation when the observation is displayed on said observation display unit (1);
a treatment information obtaining unit (3) obtaining the treatment information input in response to the notification, and
a storage unit (4) storing observation information indicating the observation associated with the treatment information.

2. The system according to claim 1, further comprising:
an observation term selection result obtaining unit obtaining a selection result of an observation term corresponding to the observation from a plurality of observation terms; and
a supplementary information result obtaining unit obtaining the selection result of supplementary information corresponding to the observation from plural pieces of supplementary information associated with the observation term relating to the selection result, wherein
said observation information is formed by the observation terms relating to the selection result about the observation term and the supplementary information relating to the selection result about the supplementary information.

3. The system according to claim 2, further comprising
an observation term list display unit displaying a list of a plurality of observation terms.

4. The system according to claim 2, further comprising
a supplementary information list display unit displaying a list of supplementary information associated with the observation term, wherein
said supplementary information result obtaining unit can obtain a selection result from the list of the supplementary information.

5. The system according to claim 4, wherein
said supplementary information list display unit displays a list of the supplementary information based on the selection result of the observation term obtained by said observation term selection result obtaining unit.

6. The system according to claim 2, wherein
said observation display unit (1) displays a list of observation terms relating to the selection result about the observation term and the supplementary information relating to the selection result about the supplementary information.

7. The system according to claim 2, further comprising
an observation text generation unit generating observation text representing the observation term relating to the selection result and the supplementary information relating to the selection result about the supplementary information, wherein
said observation display unit displays the observation text.

8. The system according to claim 7, further comprising
an observation display format switch unit switching between a display of the observation on said observation display unit represented as a list of observation terms relating to the selection result about the observation term and supplementary information relating to the selection result about the supplementary information, and a display of the observation text.

9. A system for use in generating a diagnosis report, comprising:
an observation term selection result obtaining unit obtaining a selection result from a plurality of observation terms about an observation term corresponding to observation on an examination result;
a supplementary information result obtaining unit obtaining the selection result of supplementary information corresponding to the observation from plural pieces of supplementary information associated with the observation term relating to the selection result; and
an observation information storage unit storing observation information which is formed by the observation term relating to the selection result about an observation term and the supplementary information relating to the selection result about the supplementary information, and indicates the observation.

10. A method for generating a diagnosis report, comprising:
displaying observation on an examination result on a display device;
transmitting a notification of a request to input treatment information about the treatment in response to a display of the observation;
obtaining the treatment information input in response to the notification, and
storing observation information indicating the observation associated with the treatment information.

11. A method for generating a diagnosis report, comprising:
obtaining a selection result from a plurality of observation terms about an observation term corresponding to observation on an examination result;
obtaining the selection result of supplementary information corresponding to the observation from plural pieces of supplementary information associated with the observation term relating to the selection result; and
storing observation information which is formed by the observation term relating to the selection result about an observation term and the supplementary information relating to the selection result about the supplementary information, and indicates the observation.

12. A computer-readable recording medium which stores a program used to direct a computer to generate a diagnosis report, and is executed by the computer to perform the process comprising:
an observation displaying process of allowing a display device to display observation on an examination result;
a treatment information input request notifying process of transmitting a notification of a request to input treatment information about the treatment in response to a display of the observation;
a treatment information obtaining process of obtaining the treatment information input in response to the notification, and
a storing process of storing observation information indicating the observations associated with the treatment information.

13. A computer-readable recording medium which stores a program used to direct a computer to generate a diagnosis report, and is executed by the computer to perform the process comprising:
an observation term selection result obtaining process of obtaining a selection result from a plurality of observation terms about an observation term corresponding to observation on an examination result;
a supplementary information result obtaining process of obtaining the selection result of supplementary information corresponding to the observation from plural pieces of supplementary information associated with the observation term relating to the selection result; and
an observation information storage process of storing observation information which is formed by the observation term relating to the selection result about an observation term and the supplementary information relating to the selection result about the supplementary information, and indicates the observation.
